# EUROPÄISCHE PATENTSCHRIFT

(11) **EP 0 880 530 B1**
(45) Veröffentlichungstag und Bekanntmachung des Hinweises auf die Patenterteilung: **02.07.2003**
(21) Anmeldenummer: 97903308.1
(22) Anmeldetag: 17.02.1997
(51) Int. Cl.: C07F 9/10, A61K 9/127, C07D 317/22, C07C 43/13

(54) **PHOSPHATIDYLOLIGOGLYCERINE**
PHOSPHATIDYL OLIGOGLYCEROLS
PHOSPHATIDYLOLOGOGLYCERINES

(30) Priorität: 16.02.1996 DE 19605833; 03.06.1996 DE 19622224
(43) Veröffentlichungstag der Anmeldung: 02.12.1998
(73) Patentinhaber: Max-Planck-Gesellschaft zur Förderung der Wissenschaften e.V., 80539 München (DE)
(72) Erfinder: EIBL, Hans-Jörg, D-37120 Bovenden (DE)
(74) Vertreter: Huber, Bernhard, Dipl.-Chem.
(86) Internationale Anmeldenummer: EP9700749
(87) Internationale Veröffentlichungsnummer: WO97030058

(56) Entgegenhaltungen:
- EP-A- 0 071 019
- DD-A- 240 020
- DE-A- 3 427 093
- DE-A- 3 619 883
- INT. J. PHARM. (IJPHDE,03785173);94; VOL.111 (1); PP.103-7, FACULTY OF PHARMACEUTICAL SCIENCES, TEIKYO UNIVERSITY, SAGAMIKO;KANAGAWA; 199-01; JAPAN (JP), XP000672277 MARUYAMA K ET AL: "Phosphatidyl polyglycerols prolong liposome circulation in vivo" in der Anmeldung erwähnt
- JOURNAL OF LIPOSOME RESEARCH, Bd. 2, Nr. 3, 1.Januar 1992, Seiten 289-305, XP000303894 ALLEN T M: "STEALTH LIPOSOMES: FIVE YEARS ON"
- TETRAHEDRON (TETRAB,00404020);95; VOL.51 (16); PP.4732-32, INDIAN INST. TECHNOLOGY;DEP. CHEM.; BOMBAY; 400 076; INDIA (IN), XP000674272 BAIG M H A ET AL: "Stereoselective total synthesis of (2R,2'S,3Z)-1'-O-(2-methoxy-3-he xadecenyl)glycerol and (2R,2'S)-1'-O-(2-methoxyhexadecyl)glycerol - potential antitumor compounds from shark liver oil"

## Beschreibung

Die Erfindung betrifft Phosphatidylverbindungen, die einen definierten hydrophilen Rest enthalten, sowie Liposomen, die eine verlängerte Lebensdauer aufweisen.

Herkömmliche Liposome weisen im Serum eine Verweilzeit von bis zu 5 Stunden auf. Insbesondere bei der Verwendung von Liposomen als Träger für pharmazeutische Wirkstoffe ist jedoch eine möglichst lange Verweilzeit von Liposomen im Blutkreislauf wünschenswert.

Es wurden daraufhin die sogenannten "Stealth-Liposomen" entwickelt, die eine verlängerte Lebensdauer aufweisen. Diese "Stealth-Liposomen" sind auf der Basis von Phosphatidylverbindungen aufgebaut, die einen verlängerten Polyethylenglykolrest enthalten. Der Polyethylenglykolrest erwies sich für die angestrebte verlängerte Lebensdauer am wirksamsten bei Molekulargewichten zwischen 2000 und 3000. Ein wesentlicher Nachteil dieser "Stealth-Liposomen" bzw. der Phosphatidylverbindungen mit Polyethylenglykolrest liegt allerdings darin, daß es sich nicht um exakt definierte Verbindungen handelt, da die Polyethylenglykolreste unterschiedliche Kettenlängen aufweisen.

Weiterhin wurde von Maruyama et al. (Int. J. Pharmac. 111 (1994), 103-107) die Verwendung von Dipalmitoylphosphatidylpolyglycerinen zur Verlängerung der Liposomenzirkulation vorgeschlagen. Da jedoch von technischen Polyglycerinen ausgegangen wurde, wurden auch hier keine einheitlichen Produkte erhalten. Die technischen Polyglycerine, die aus einem Gemisch aus Polyglycerinen verschiedener Kettenlänge und Monoglycerin bestehen und durch ihr mittleres Molekulargewicht charakterisiert sind, wurden mittels Phospholipase D phosphatidyliert. Mit den so erhaltenen Produkten konnte jedoch nur eine geringe Erhöhung der Lebensdauer von Liposomen in Blut beobachtet werden.

Aus DD-A-240020 ist ein Verfahren zur Herstellung pharmakologisch wirksamer Phospholipide bekannt. Diesen Verbindungen fehlt jedoch der definierte hydrophile Rest, der für die Verbindungen der Erfindung charakteristisch ist. Mit Liposomen befasst sich diese Veröffentlichung nicht.

Aufgabe der vorliegenden Erfindung war die Bereitstellung von Verbindungen, die die Lebensdauer von Liposomen erhöhen und zugleich eine exakt angebbare Zusammensetzung aufweisen.

Die Aufgabe wird erfindungsgemäß gelöst durch eine Verbindung einer allgemeinen Formel (A) 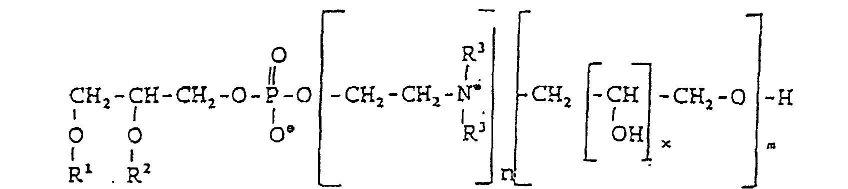 worin R¹ und R² unabhängig voneinander Wasserstoff, einen gesättigten oder ungesättigten Alkyl- oder Acylrest darstellen, der gegebenenfalls verzweigt oder/und substituiert sein kann, R³ Wasserstoff oder einen Alkylrest darstellt,
- n: = 0 oder 1 ist,
- x: eine ganze Zahl von 1 bis 4 darstellt und
- m: eine ganze Zahl von 2 bis 10, falls n = 0, oder eine ganze Zahl von 1 bis 10, falls n = 1, darstellt sowie 1 ist, falls x größer als 1 ist,
wobei im Falle n = 0 die Verbindung hinsichtlich des Werts von m größer als 90 % einheitlich ist, ausgenommen Verbindungen mit R¹ = Hexadecyl, R² = Methyl, n' = 1, x = 1 und m = 1.
Der dieser Erfindung zugrundeliegende schrittweise Aufbau der hydrophilen Reste der Phosphatidylverbindungen der Formel (A) ermöglicht es, eine genau definierte Zusammensetzung der Verbindungen zu erhalten.

Es handelt sich also bei der erfindungsgemäßen Verbindung der Formel (A) nicht um ein Gemisch verschiedener Moleküle unbestimmter Zusammensetzung und Kettenlänge, sondern es kann gezielt eine gewünschte Struktur erhalten werden. Dies bedeutet, daß beispielsweise, falls das gewünschte Produkt ein Triglycerinderivat ist, also x = 1 und m = 3 in Formel (A), der Gehalt an Monoglycerin-, Diglycerin-, Tetraglycerin- und der Gehalt an Monoglycerin-, Diglycerin-, Tetraglycerin- und höheren Oligoglycerinderivaten gering ist. Bevorzugt wird ein Glycerinderivat bestimmter Kettenlänge im wesentlichen frei von Glycerinderivaten anderer Kettenlänge erhalten. Insbesondere ist der Gehalt an Monoglycerinderivaten gering und beträgt weniger als 5 %, bevorzugt weniger als 1 % und besonders bevorzugt weniger als 0,1 %, bezogen auf das gewünschte Oligoglycerinderivat.

Erfindungsgemäß stellt die Verbindung der Formel (A) eine einheitliche Verbindung definierter Struktur dar. Bevorzugt ist die Verbindung hinsichtlich des Werts von m größer als 95 %, besonders bevorzugt größer als 99 % einheitlich. Es ist jedoch auch möglich, die Verbindung mit einer Einheitlichkeit von mehr als 99,9 % hinsichtlich des Werts von m bereitzustellen.

Bevorzugt handelt es sich bei der Verbindung um Oligoglycerinderivate mit 2 bis 5 Glycerineinheiten, bevorzugt mit 2 bis 4 Glycerineinheiten. Es handelt sich dabei bevorzugt um 1.3-verknüpfte lineare Oligoglycerinreste.

Die Reste R¹ und R² stellen erfindungsgemäß bevorzugt unabhängig voneinander Wasserstoff, einen gesättigten oder ungesättigten C₁-C₂₄-Alkyl- oder C₁-C₂₄-Acylrest, bevorzugt Wasserstoff oder einen gesättigten oder ungesättigten C₈-C₂₄-Alkyl- oder C₈-C₂₄-Acylrest dar, wobei bevorzugt mindestens einer der Reste R¹ und R² einen Acylrest darstellt.

Der Rest R³ stellt bevorzugt Wasserstoff oder einen Alkylrest mit 1 bis 4 Kohlenstoffatomen dar.

Die Verbindung der Formel (A) kann als Racemat, das eine Phospho-rac-(1 oder 3)-oligoglycerinverknüpfung enthält oder als stereospezifisches Isomer vorliegen. Die Stereoisomere können eine Phospho-sn-1-oligoglycerinverknüpfung oder eine Phosphosn-3-oligoglycerinverknüpfung aufweisen. Die Bildung der stereospezifischen Verknüpfung kann analog zu den in der Literatur beschriebenen Verfahren durchgeführt werden (DE 31 30 867 A1; H. Eibl et al., Chem. Phys. Lipids, 28 (1981), 1- 5, 41 (1986), 53-63 und 47 (1988, 47-53).

Ein weiterer Gegenstand der Erfindung sind Liposomen, die Phospholipide oder/und Alkylphospholipide, gegebenenfalls Cholesterin und 1 bis 50 Mol-% einer Verbindung der allgemeinen Formel (A) 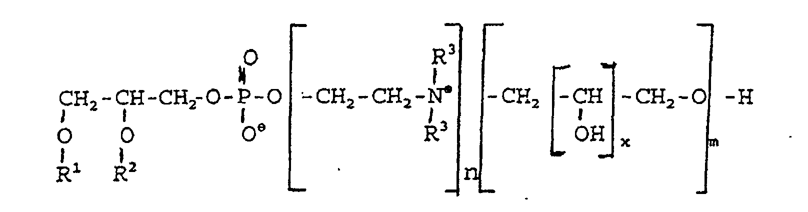 oder deren Salze enthalten, wobei das Cholesterin, die Phospholipide, die Alkylphospholipide und die Verbindung der Formel (A) zusammen 100 Mol-% ergeben, worin R¹ und R² unabhängig voneinander Wasserstoff, einen gesättigten oder ungesättigten Alkyl- oder Acylrest darstellen, der gegebenenfalls verzweigt oder/und substituiert sein kann,
R³ Wasserstoff oder einen Alkylrest darstellt,
- n: = 0 oder 1 ist,
- x: eine ganze Zahl von 1 bis 4 darstellt und
- m: eine ganze Zahl von 2 bis 10, falls n = 0, oder eine ganze Zanl von 1 bis 10, falls n = 1, darstellt sowie 1 ist, falls x größer als 1 ist, wobei im Falle n = 0 die Verbindung (A) hinsichtlich des Werts von m größer als 90 % einheitlich ist.

Die erfindungsgemäßen Liposomen weisen eine Halbwertszeit im Serum von bis zu 18 bis 20 Stunden auf. Dabei wurde überraschenderweise ein linearer Abfall der Liposomenkonzentration im Blut gefunden.

Erfindungsgemäß bevorzugt wird die Verbindung (A) mit einer Einheitlichkeit von größer als 95 %, besonders bevorzugt größer als 99 % hinsichtlich des Werts von m verwendet. Es ist aber auch möglich, die Verbindung (A) in praktisch reiner Form worin R¹ und R² unabhängig voneinander Wasserstoff, einen gesättigten oder ungesättigten Alkyl- oder Acylrest darstellen, der gegebenenfalls verzweigt oder/und substituiert sein kann, R³ Wasserstoff oder einen Alkylrest darstellt,
- n: = 0 oder 1 ist,
- x: eine ganze Zahl von 1 bis 4 darstellt und
- m: eine ganze Zahl von 2 bis 10, falls n = 0, oder eine ganze Zahl von 1 bis 10, falls n = 1, darstellt sowie 1 ist, falls x größer als 1 ist,
wobei im Falle n = 0 die Verbindung hinsichtlich des Wertes von m größer als 90 % einheitlich ist,
**dadurch gekennzeichnet,**
dass ein definiertes Oligoglycerin oder ein C₄-C₆-Zuckeralkohol unter Verwendung eines Phosphorylierungsmittels an einen Alkohol der Formel CH₂OR¹-CHOR²-CHOH angehängt wird. 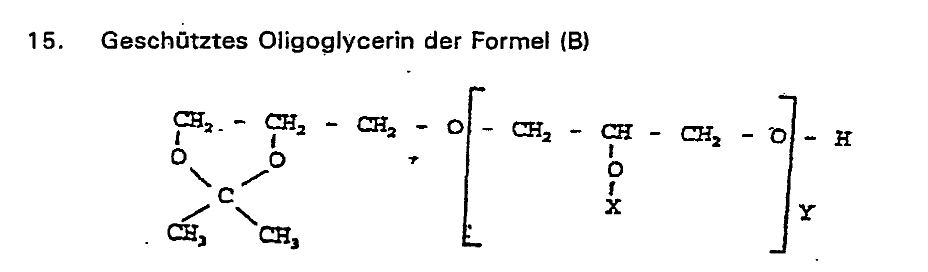 wörin Y eine ganze Zahl von 1 bis 9 und X eine Benzyl-, Alkyl- oder Tetrahydropyranylgruppe darstellt, ausgenommen X = Methyl, wenn Y = 1 ist. 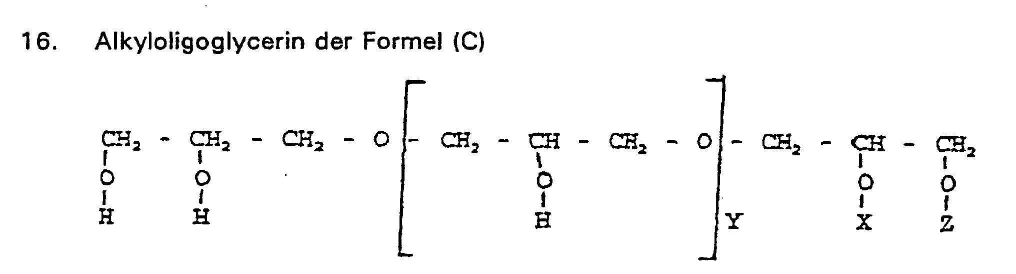 und Phospholipide oder/und Alkylphospholipide bevorzugt. Im Falle einer negativen Überschußladung besteht eine bevorzugte, Zusammensetzung der Liposomen aus 0 bis 70 Mol-% Cholesterin, 1 bis 15 Mol-% einer Verbindung der Formel (A) sowie Phospholipiden oder/und Alkylphospholipiden. Ein höherer Anteil an Verbindungen der Formel (A) mit negativer Überschußladung würde zur Instabilität der Liposomen im Blutkreislauf führen. Bevorzugt umfassen die Liposomen 35 bis 43 Mol-%, besonders bevorzugt 38 bis 42 Mol-% Cholesterin, 5 bis 15 Mol-% einer Verbindung der Formel (A) und Phospholipide oder/und Alkylphospholipide.

Die Phospholipide oder/und Alkylphospholipide können beispielsweise Diacylphosphoglycerine definierter Struktur sein. Allgemein können diese Bestandteile der Lipide als Verbindungen definierter Struktur eingesetzt werden.

Im Falle x > 1 stammt der Rest -CH₂(-CHOH)ₓ-CH₂-OH bevorzugt von Zuckeralkoholen, die für x = 2,4 Hydroxylgruppen, für x = 3,5 Hydroxylgruppen und für x = 4,6 Hydroxylgruppen aufweisen. Beispiele solcher Reste sind Mannitderivate für x = 4, Lyxitderivat für x = 3 und Threitderivate für x = 2.

Die erfindungsgemäßen Liposomen weisen eine deutlich erhöhte Halbwertszeit im Blutkreislauf auf. Bevorzugt beträgt ihre Halbwertszeit mindestens 10 Stunden, besonders bevorzugt mehr, als 12 Stunden. Es wurden Halbwertszeiten der erfindungsgemäßen Liposomen von 18 bis 20 Stunden erhalten. Überraschenderweise wurden dabei absolut lineare Abfallkurven der Lipidkonzentrationen im Blut festgestellt. Erfindungsgemäß bevorzugt befinden sich nach 6 Stunden mehr als 50 % und besonders bevorzugt mehr als 60 % der zugegebenen Liposomenmenge im Blut.

Als besonders überraschende Eigenschaft der erfindungsgemäßen Liposomen hat sich ihre bevorzugte Anreicherung in der Milz herausgestellt. In Abhängigkeit von der Zusammensetzung und der Größe der Liposomen wurden bereits Anreicherungsfaktoren in der Milz im Vergleich zur Leber um etwa das 25-fache erreicht. Dabei steigt die Anreicherung in der Milz verglichen mit der Leber mit zunehmendem Wert für m in Formel A und mit zunehmender Größe der Liposomen. So steigt der Anreicherungsgrad in der Milz beim Übergang von SUVS (Small Unilamellar Liposomes; Durchmesser etwa 60 nm) auf LUVs (Large Unilamellar Liposomes; Durchmesser etwa 190 nm) um ein Vielfaches. Die bevorzugte Anreicherung in der Milz nimmt auch mit zunehmender Zahl der Kohlenstoffatome in R¹ und R² zu.

Darüber hinaus wurde gefunden, daß die erfindungsgemäßen Liposomen auch in bestimmten Tumorgeweben angereichert werden. So wurde gefunden, daß eine solche Anreicherung bei dem mit Methylnitrosoharnstoff induzierten Mammakarzinom (MNU-Karzinom) stattfindet.

Weiterhin können die erfindungsgemäßen Liposomen zusätzlich einen oder mehrere pharmazeutische Wirkstoffe enthalten.

Als Wirkstoffe können in der Regel alle Wirkstoffe verwendet werden, die sich mittels Liposomen überhaupt ins Plasma einbringen lassen. Bevorzugte Wirkstoffgruppen sind einerseits Cytostatika, insbesondere Anthracyclin-Antibiotika, wie etwa Doxorubicin, Epirubicin oder Daunomycin, wobei Doxorubicin besonders bevorzugt ist. Weitere bevorzugte Cytostatika sind Idarubicin, Hexadecylphosphocholin, 1-Octadecyl-2-methyl-racglycero-3-phosphocholin, 5-Fluoruracil, cis-Platinkomplexe wie Carboplatin und Novantron sowie Mitomycine.

Weitere bevorzugte Wirkstoffgruppen sind immunmodulierende Substanzen wie etwa Cytokine, wobei unter diesen wiederum die Interferone und insbesondere das α-Interferon besonders bevorzugt sind, antimykotisch wirksame Substanzen (z.B. Amphotericin B) und Wirkstoffe gegen Protozoenerkrankungen (Malaria, Trypanosomen- und Leishmanien-Infektionen). Ebenfalls bevorzugt ist Taxol als Wirkstoff.

Eine weitere bevorzugte Wirkstoffgruppe sind lytische Wirkstoffe, wie sie in der DE 41 32 345 A1 beschrieben sind. Der Inhalt dieser Patentanmeldung wird hiermit durch Bezugnahme aufgenommen. Bevorzugt sind Miltefosin, Edelfosin, Ilmofosin sowie SRI62-834.

Ein weiterer Gegenstand der vorliegenden Erfindung ist somit die Verwendung von erfindungsgemäßen Liposomen zur Herstellung eines Antitumormittels, wobei der Wirkstoff besonders bevorzugt Doxorubicin ist.

Ein weiterer Gegenstand der vorliegenden Erfindung ist die Verwendung der erfindungsgemäßen Liposomen zur Herstellung eines Mittels zur Beeinflussung der Zellproliferation, wobei der Wirkstoff ein Cytokin, besonders bevorzugt α-Interferon ist.

Ein weiterer Gegenstand der Erfindung ist eine pharmazeutische Zusammensetzung, die die oben beschriebenen Liposomen und in den Liposomen eingeschlossen einen oder mehrere pharmazeutische Wirkstoffe gegebenenfalls zusammen mit pharmazeutisch üblichen Verdünnungs-, Hilfs-, Träger- und Füllstoffen enthält.

Die erfindungsgemäßen Liposomen werden nach an sich bekannten Methoden hergestellt mit hierfür gängigen Vorrichtungen. Typischerweise kann eine die verschiedenen Komponenten des Liposoms einschließlich 1 bis 50 Mol-% einer Verbindung der Formel (A) enthaltende Lösung in eine Lipidsuspension überführt werden, die dann unter hohem Druck durch Düsen oder durch Lochscheiben gepreßt wird, wobei durch die Größe der Öffnungen in der Lochscheibe die Größe der erhaltenen Liposomen geregelt werden kann. Geeigente Maßnahmen zur Überführung einer Lipidsuspension in Liposomen sind dem Fachmann bekannt. Bevorzugt werden 5 bis 15 Mol-% einer Verbindung der allgemeinen Formel (A) mit 35 bis 43 Mol-% Cholesterin und 42 bis 60 Mol-% Phospholipiden oder/und Alkylphospholipiden in eine Lipidsuspension überführt, die dann durch geeignete Maßnahmen auf an sich bekannte Weise in Liposomen überführt wird.

Solch bekannte Verfahren können auch zur Herstellung einer pharmazeutischen Zubereitung, die die erfindungsgemäßen Liposomen und einen oder mehrere pharmazeutische Wirkstoffe enthält, verwendet werden. Zum Einschluß wasserunlöslicher Wirkstoffe wird der Wirkstoff dabei zusammen mit den Lipidbestandteilen gelöst, während zum Einschluß wasserlöslicher Wirkstoffe der Lipidfilm mit einer wäßrigen Lösung versetzt wird, die den wasserlöslichen Wirkstoff enthält.

Die erfindungsgemäßen Verbindungen der Formel (A) können für n = 1 durch Verknüpfen eines definierten Oligoglycerins über die Aminogruppe mit einem Phosphatidylethanolamin hergestellt werden. Dabei erhält man neutrale Verbindungen, also Verbindungen ohne Überschußladung. Bei den zur Verknüpfung eingesetzten definierten Oligoglycerinen handelt es sich um Verbindungen der Formel (B).

Zur Herstellung von Verbindungen der allgemeinen Formel (A), in der n = 0 ist, wird ein definiertes Oligoglycerin mit einem Phosphatidylglycerin verknüpft. Weiterhin können Verbindungen der allgemeinen Formel (A) mit n = 0 dadurch hergestellt werden, daß ein definiertes Oligoglycerin oder ein C₄-C₆-Zuckeralkohol unter Verwendung eines Phosphorylierungsmittels mit einem Alkohol der Formel CH₂-OR¹-CHOR²-CHOH verknüpft wird. Bevorzugt wird als Phosphorylierungsmittel POCl₃ verwendet.

Die Herstellung von Phospholipiden aus Diacylglycerinen ist in der Literatur beschrieben (DE 32 39 817 A1; P. Woolley et al., Chem. Phys. Lipids 47 (1988), 55-62; H. Eibl et al., Chem. Phys. Lipids 47 (1988), 63-68) und kann entsprechend angewendet werden.

Durch die oben beschriebenen Verfahren kann eine racemische Verbindung gebildet werden, die eine Phospho-rac-(1 oder 3)-oligoglycerinverknüpfung enthält. Bevorzugt werden durch die

Durch die oben beschriebenen Verfahren kann eine racemische Verbindung gebildet werden, die eine Phospho-rac-(1 oder 3)-oligoglycerinverknüpfung enthält. Bevorzugt werden durch die Verfahren stereospezifische Verbindungen gebildet, die eine Phopsho-sn-1-oligoglycerinverknüpfung oder eine Phopsho-sn-3-oligoglycerinverknüpfung aufweist. Bevorzugt wird zur Herstellung einer Verbindung der Formel (A) ein lineares Oligoglycerin definierter Kettenlänge eingesetzt.

Ein weiterer Gegenstand der Erfindung ist ein geschütztes Oligoglycerin der Formel (B), 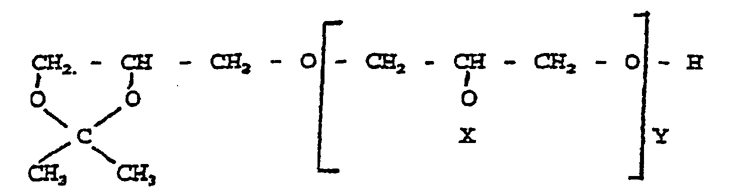 worin Y eine ganze Zahl von 1 bis 9 und X eine Benzyl-, Alkyloder Tetrahydropyranylgruppe darstellt, ausgenommen X = Methyl, wenn Y = 1 (bekannt aus Tetrahedron, 1995, 51(16), 4723-4732, Verbindung 11). Bevorzugt ist Y eine ganze Zahl von 1 bis 3. Erfindungsgemäß gelingt es, 1.3-verknüpfte oligoglycerine in praktisch reiner Form zu erhalten. Es können Oligoglycerine einer bestimmten Kettenlänge hergestellt werden, die praktisch keine Verunreinigungen durch Oligoglycerine anderer Kettenlänge entbalten. Zudem weisen die erfindungsgemäß verwendeten oligoglycerine praktisch keine Verunreinigung durch monomeres Glycerin auf. Es handelt sich also um einheitliche Verbindungen definierter Struktur.

In dem Oligoglycerin kann X auch eine andere geeignete Schutzgruppe darstellen. Weiterhin kann anstelle von Aceton auch eine andere Schutzgruppe, insbesondere ein anderes Keton vorhanden sein.

Weiterhin umfaßt die Erfindung alkyloligoglycerine der Formel (C) 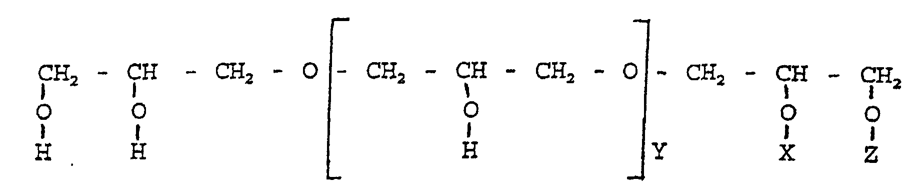 worin Y eine ganze Zahl von 0 bis 8, bevorzugt eine ganze Zahl von 1 bis 3 darstellt und einer der Reste X oder Z einen gesättigten oder ungesättigten Alkylrest und der andere der Reste Wasserstoff darstellt , ausgenommen Y = 0, wenn X = Methyl (bekannt aus Tetrahedron, 1995, 51(16), 4723-4732, Verbindung 10) oder einer der Reste X oder Z C₁₆H₃₃ ist (bekannt aus DE-A-36 19 883, Seite 33). Auch bei den Alkyloligoglycerinen handelt es sich um einheitliche Verbindungen definierter Struktur.

Die Herstellung von Oligoglycerinen, geschützten Oligoglycerinen und Alkyloligoglycerinen ist von besonderem Interesse, weil mit Hilfe dieser Ausgangsstoffe eine Reihe von wichtigen und neuen Hilfsstoffen zur Lösungsvermittlung und zur Verbesserung der Membranpermeation erhalten werden können. Von besonderem Interesse zur Verlängerung der Liposomenlebenszeit im Blut ist die Herstellung von Phosphatidyloligoglycerinderivaten der Formel (A), die zusätzliche Hydroxylgruppen im polaren Bereich tragen.

Aufgrund der bevorzugten Anreicherung der erfindungsgemäßen Liposomen in der Milz eignen sich diese Liposomen generell für die selektive Einführung von Substanzen in die Milz. Hierbei kann es sich um Arzneimittel, Kontrastmittel oder dgl. handeln. Insbesondere ist dies aber für die Verbesserung der Qualität von Impfstoffen von Bedeutung, da die Milz für die Bildung von Antikörpern im Rahmen des Immunsystems besonders wichtig ist. Ebenso ist die festgestellte Anreicherung der erfindungsgemäßen Liposomen in Tumorgewebe von Bedeutung für die spezifische Zufuhr von Wirkstoffen, Kontrastmitteln und dgl. in ein solches Tumorgewebe.

Die Erfindung wird durch die folgenden Beispiele in Verbindung mit den beigefügten Zeichnungen näher erläutert. In den Zeichnungen stellen dar:
Figur 1 eine graphische Darstellung, welche die Verteilung der erfindungsgemäßen Liposomen in Milz und Leber pro Gesamtorgan darstellt.
Figur 2 eine graphische Darstellung, welche die Verteilung der erfindungsgemäßen Liposomen in Milz und Leber pro Gramm Organ darstellt.

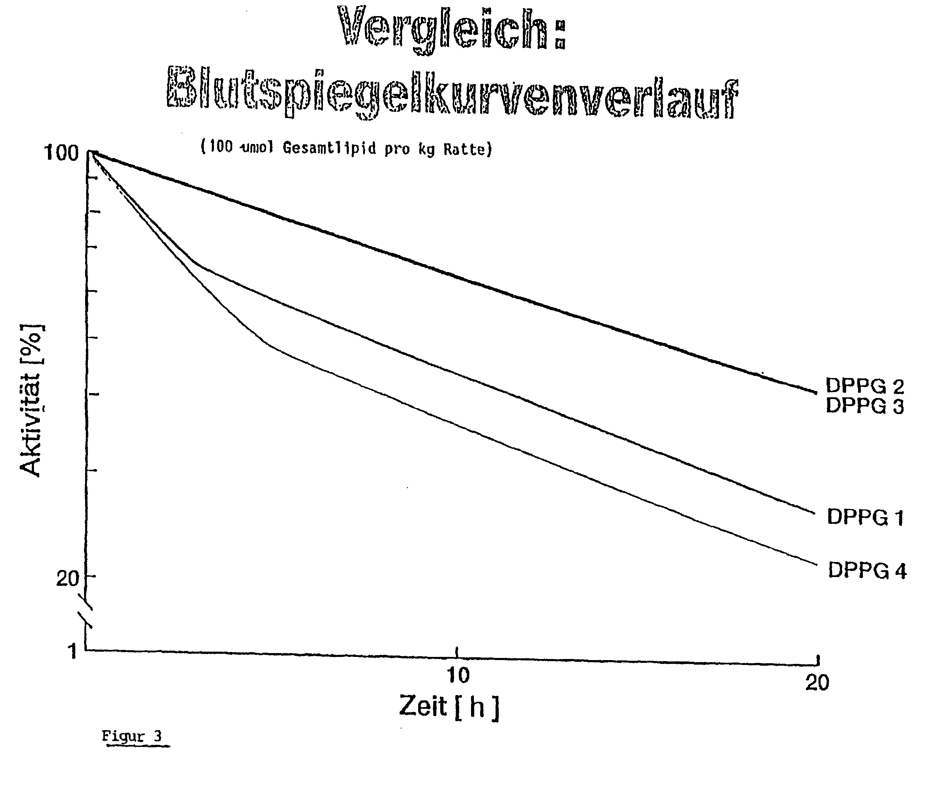

Figur 3 eine graphische Darstellung, welche den Blutspiegelkurvenverlauf in Abhängigkeit von der Zeit für verschiedene erfindungsgemäße Liposomen darstellt.

### Beispiel 1

In einem Tierversuch wurden Liposomen verwendet, die aus 40 Mol-% Cholesterin, 10 Mol-% Phosphatidylglycerin und 50 % Dipalmitoyllecithin bestanden. Dabei ergab sich eine Halbwertszeit im Serum von 4 Stunden mit einer typischen Abklingkurve, d.h. anfangs sehr rasche Abnahme, später langsamere Abnahme.

Es wurden Liposomen der gleichen Zusammensetzung hergestellt, in denen das Phosphatidylglycerin durch ein erfindungsgemäßes Phosphatidylglycerin G2 ersetzt. Es wurde eine Halbwertszeit im Serum von 18 bis 20 Stunden mit absolut linearer Abfallkurve erhalten. Die lineare Abfallkurve wurde unabhängig von der Größe der hergestellten Liposomen gefunden. Es wurde mit 50 nm Liposomen und mit 150 nm Liposomen der gleiche lineare Abfall der Liposomkonzentration im Serum gefunden. Weiterhin wurde die lineare Abnahme der Liposomenkonzentration im Blut bei verschiedenen Anfangskonzentrationen festgestellt.

### Beispiel 2

### Prozentualer Anteil der Liposomen in der Blutzirkulation nach 6 Stunden

Es wurden erfindungsgemäße Liposomen aus Dipalmitoyl-sn-G-3-PC/Cholesterin/Dipalmitoyl-sn-G-3-PG_{Y} im molaren Verhältnis von 45 : 45 : 10 hergestellt. Der prozentuale Anteil der ursprünglich zugegebenen Liposomenmenge, die nach 6 Stunden im Blut gefunden wurden, sind in Tabelle 1 angegeben. Zum Vergleich sind die von Maruyama et al. unter den gleichen Bedingungen für das System Distearyl-sn-G-3-PC/Cholesterin/Dipalmitoyl-sn-G-3-PG_{Yi} 45 : 45 : 10 erhaltenen Werte angegeben. Es zeigt sich eine deutliche Erhöhung der gefundenen Liposomenmenge im Vergleich zum Stand der Technik.

**Tabelle 1:**

| Y | Vergleichsbeispiel | Y | Beispiel der Erfindung |
|---|---|---|---|
| 0 | 18 % | 0 | 21 % |
| 2 | 19 % | 2 | 80 % |
| 3 | - | 3 | 82 % |
| 4 | 20 % | 4 | 56 % |

### Beispiel 3

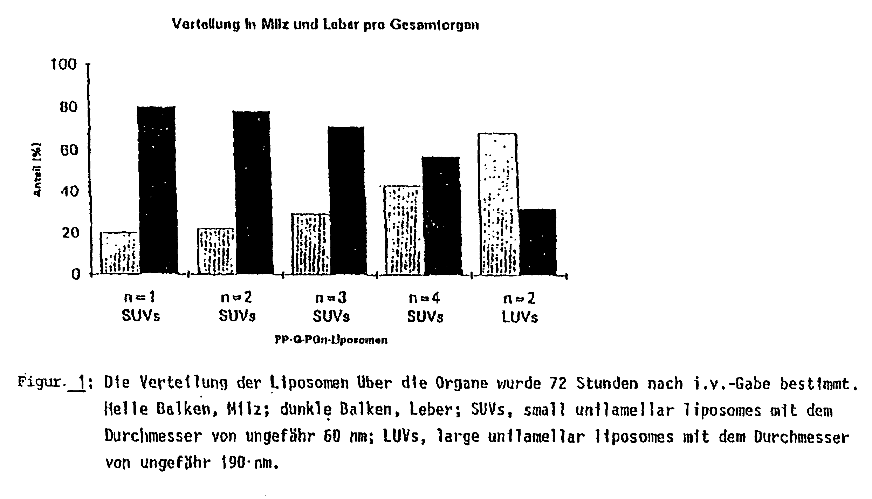

Liposomen aus 1,2-Dipalmitoyl-sn-glycero-3-phosphocholin, 1,2-Dipalmitoyl-sn-glycero-3-phosphoglyceroglycerin (PGₙ) und Cholesterin im molaren Verhältnis 4:1:5 wurden mit tritiummarkiertem Inulin dotiert. Diese Liposomen wurden in einer Dosierung von 100 µmol Lipid pro kg Ratte an Ratten verabreicht und nach 72 Stunden die Verteilung dieser Liposomen über die Organe Milz und Leber durch Messung der Radioaktivität bestimmt. Die Organgewichte der Leber variierten zwischen 9 und 10 g, die der Milz zwischen 0,6 und 0,7 g. Figur 1 der beigefügten Zeichnung zeigt, daß bei einem etwa 15-fach höheren Lebergewicht bei Verwendung von SUVs die Verteilung mit zusätzlicher Zahl der Glycerineinheiten (x = 1; m = 1 bis 4 in Formel A) zugunsten der Milz erheblich zunimmt.

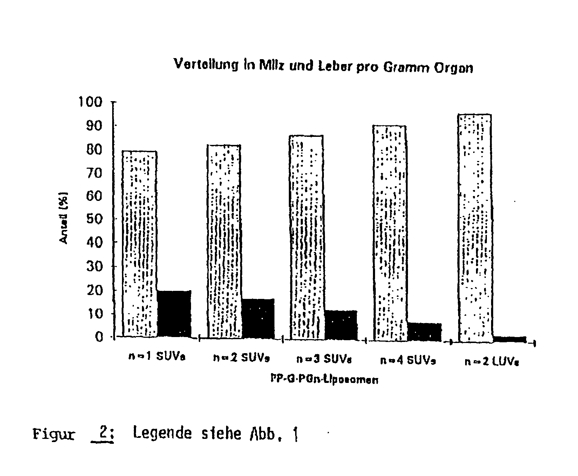

In Figur 2 ist die Aufnahme der Liposomen in die Organe Milz und Leber pro Gramm Organ wiedergegeben. Danach enthält die Milz bei n = 4 eine etwa 9 mal so hohe Konzentration an Liposomen wie die Leber, für m = 1 ist der Anreicherungsfaktor gleich 4. Die Figuren 1 und 2 zeigen zudem in der letzten Spalte den Effekt der Liposomengröße. Für LUVs mit einem Durchmesser von 190 nm ergibt sich daraus eine weitere Verstärkung der Anreicherung in der Milz, so daß bereits bei n = 2 der Anreicherungsfaktor gleich 24 ist. Praktisch wird mit diesen Liposomen das Organ Leber nicht mehr erreicht.

### Beispiel 4

### Herstellung von Verbindungen der Formel (A)

### Beispiel 4a: Zentrales Zwischenprodukt der Formel I

Die Oligoglycerine Diglycerin (G₂), Triglycerin (G₃) und Tetraglycerin (G₄) können aus einem leicht erreichbaren, zentralen Zwischenprodukt der Formel I, 1.2-Isopropyliden-rac-glycero-3.1-rac-glycero-3-allylether, hergestellt werden (siehe Schema A). 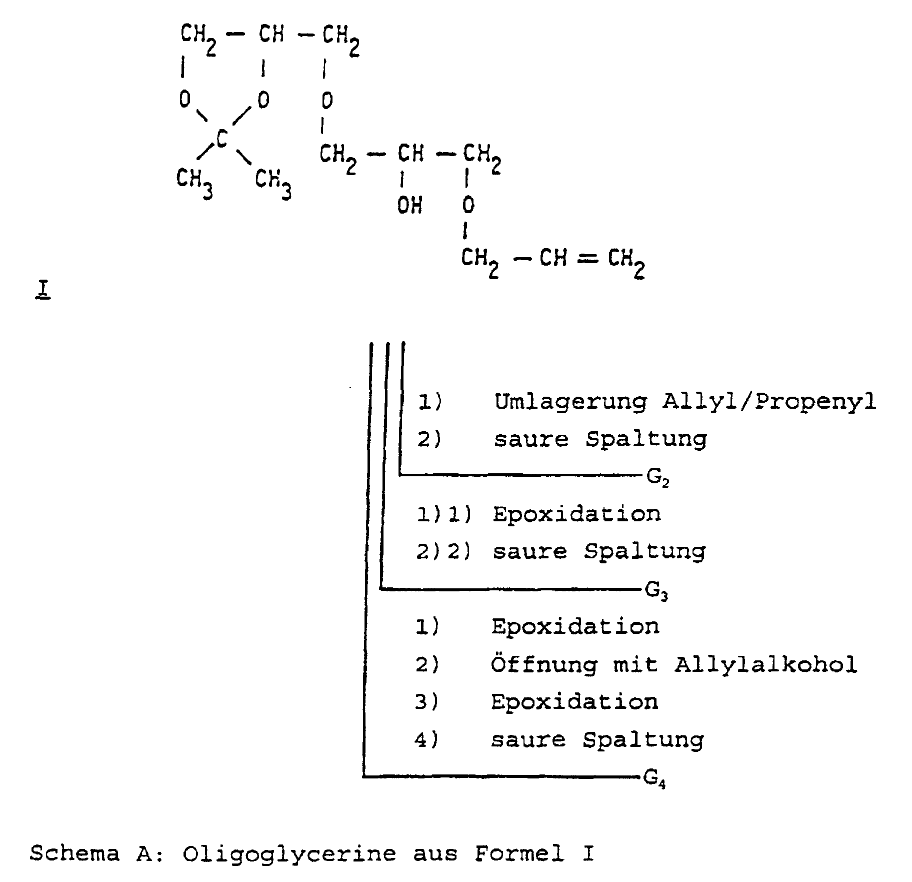 Schema A: Oligoglycerine aus Formel I

Das in Formel I beschriebene Zwischenprodukt kann in großen Mengen aus käuflichem Allylglycidylether durch NaOH katalysierte Ringöffnung mit ebenfalls im Chemikalienhandel erhältlichem 1.2-Isopropyliden-rac-glycerin erhalten werden:

### Epoxidöffnung mit Alkoholen (allgemeines Beispiel)

### Herstellung des zentralen Zwischenproduktes der Formel I: 1.2-Isopropyliden-rac-G₁-3.1-0.0-3-0-allyl-rac-G₂

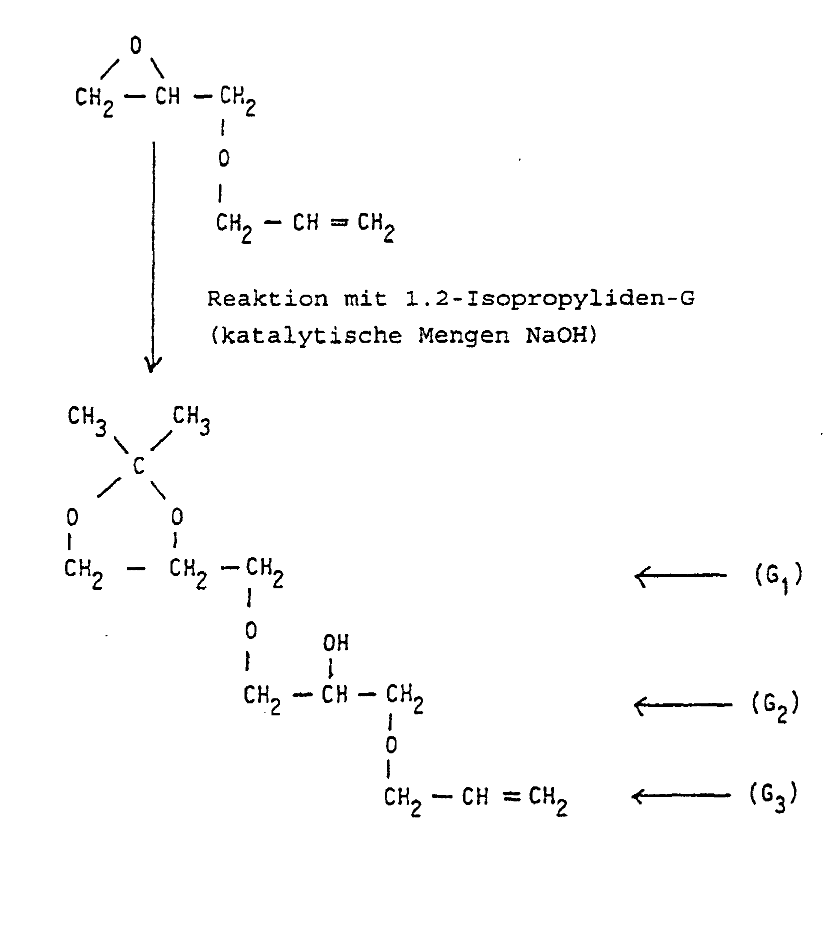

1.2-Isopropyliden-rac-glycerin (MG 132.16; 16 Mol - 2115 g) wird mit katalytischen Mengen NaOH (MG 40.00; 0.6 Mol - 24 g) versetzt und unter Rühren und Erwärmen auf 80°C in Lösung gebracht. Bei 80°C wird Allyl-glycidyl-ether (MG 114.14; 6 Mol - 685 g) tropfenweise in einem Zeitraum von 2 Stunden zugegeben und der Ansatz noch weitere 2 Stunden bei 80°C gerührt. Zu diesem Zeitpunkt hat sich das Epoxid vollständig (Rf 0.8 in Ether) unter Bildung des G₃-Bausteines (Rf 0.60 in Ether) umgesetzt. Das überschüssige Isopropyliden-rac-glycerin hat einen Rf-Wert von 0.65 in Ether und wird bei 75°C / 10 mbar aus dem Reaktionsgemisch entfernt. Der Rückstand wird mit 1 l Diisopropylether versetzt und zweimal mit jeweils 1 l NaCl (1 %-ige Lösung in H₂O) extrahiert. Die organische Phase wird einrotiert und destilliert (Kpi₁₀-1 mbar 125°C).

Die Ausbeute an reinem Produkt, 1.2-Isopropyliden-rac-G₁-3.1-0.0-3-0-allyl-rac-G₂. (MG 246.30), ist 1025 g (ca. 70 %).

Anstelle von 1.2-Isopropyliden-rac-glycerin können andere primäre Alkohole sowie Allylalkohol und Benzylalkohol nach den angegebenen Bedingungen umgesetzt werden. Entsprechend können auch andere Epoxide auf die gleiche Weise eingesetzt werden.

Das Zwischenprodukt der Formel I kann auch aus 1.2-Isopropyliden-rac-glycero-3-glycidylether durch NaOH katalysierte Ringöffnung mit Allylalkohol erhalten werden. In diesem Falle muß zunächst 1.2-Isopropyliden-rac-glycero-3-glycidylether aus Allylglycerin hergestellt werden.

### Beispiel 4b:

### Alkylierung von primären oder sekundären Hydroxylgruppen (allgemeines Beispiel)

### Herstellung eines zentralen Zwischenproduktes: 1.2-Isopropyliden-rac-G₁-3.1-0.0-2-0-benzyl-3-0-allyl-rac-G₂

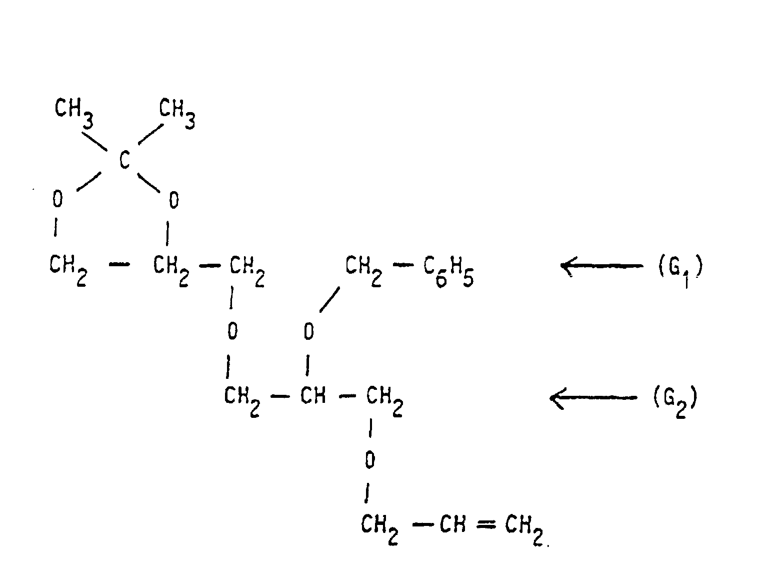

Das zentrale Zwischenprodukt, 1.2-Isopropyliden-rac-G₁-3.1-rac-G₂-0-allylether (MG 246.30; 0.5 Mol - 123 g) wird in 500 ml Tetrahydrofuran gelöst, mit Benzylchlorid (0.6 Mol - 76 g) versetzt und unter Rückfluß gekocht. Man läßt K-tert. Butylat (0.7 Mol - 79 g) gelöst in 500 ml Tetrahydrofuran langsam eintropfen. Die Reaktion ist nach 30 Minuten Kochen unter Rückfluß abgeschlossen (DC-Kontrolle - Rf-Werte in Ethylether: Edukt, Rf = 0.1; Produkt, RF = 0.4). Das Reaktionsgemisch wird mit 1 l Diisopropylether und mit 1 l 1%-iger NaCl-Lösung versetzt, geschüttelt, und die obere Phase einrotiert. Das Produkt kann direkt weiterverwendet werden oder durch Chromatographie an Kieselgel in reiner Form in ca. 90%-iger Ausbeute gewonnen werden.

| Summenformel C₁₉H₂₈O₅ (MG 336.42) | | | |
|---|---|---|---|
| ber. | C, 67.83; | H, 8.39; | O, 23.79 |
| gef. | C, 67.78; | H, 8.34; | O, - |

Anstelle von Benzylchlorid können auch Benzylbromid, Allylchlorid oder Allylbromid sowie die Halogenide oder Mesylate von primären Alkoholen verwendet werden. Insbesondere führen die Produkte aus der Reaktion primärer oder sekundärer Hydroxylgruppen mit Alkylmesylaten in hohen Ausbeuten (> 90%) zu den gewünschten Zielverbindungen.

### Beispiel 4c:

### Synthesesequenz O-Allylether → 0-Propenylether → Alkohol (allgemeines Beispiel)

### Herstellung von 2-0-Benzyl-rac-G₁-1.3-0.0-1.2-isopropyliden-rac-G₂

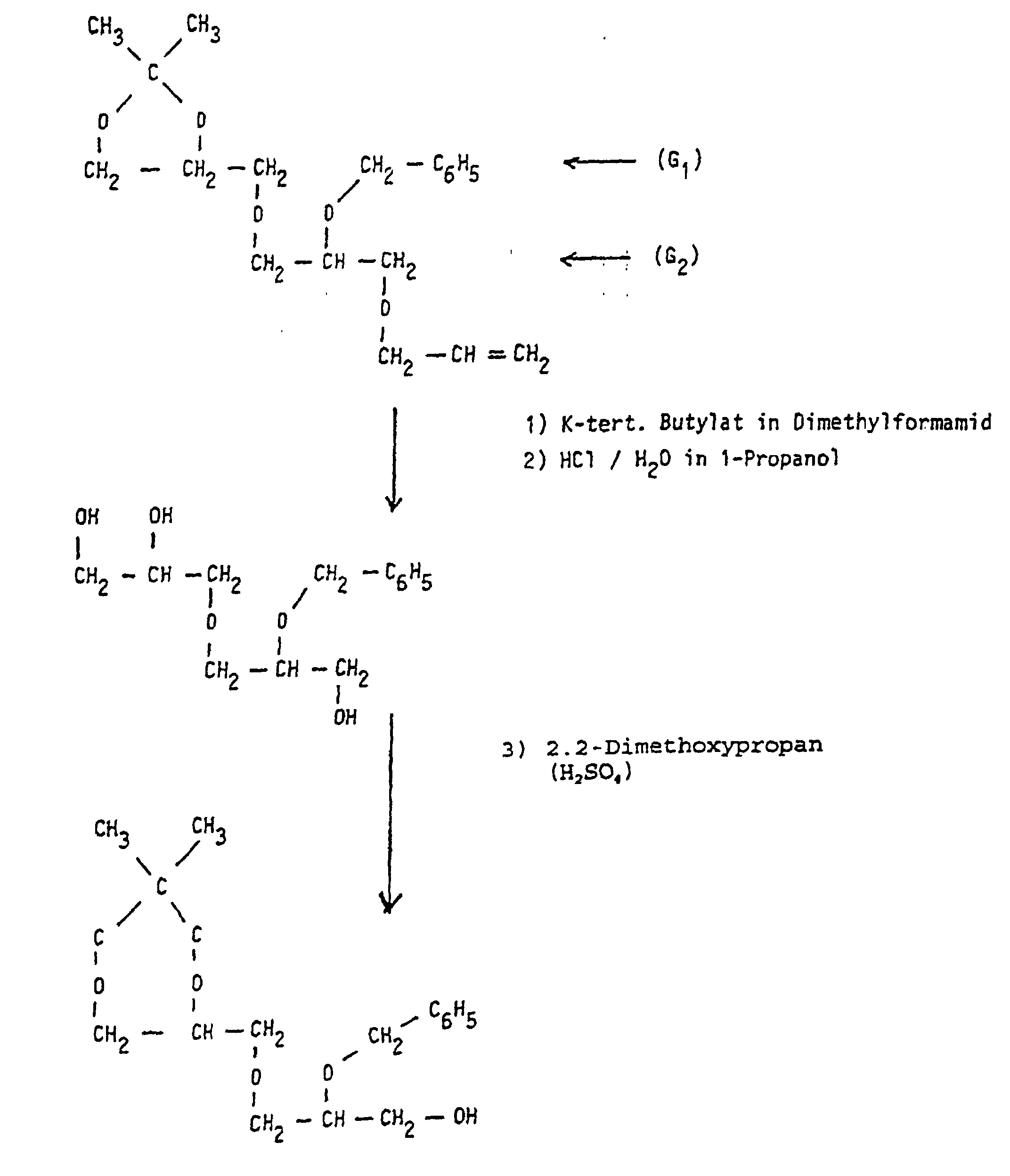

### Umlagerung

1.2-Isopropyliden-rac-G₁-3.1-0.0-2-0-benzyl-3-0-allyl-rac-G₂ (0.5 Mol - 168 g) wird in 500 ml DMF gelöst und mit K-tert. s Butylat (0.7 Mol - 79 g) versetzt. Man erhitzt unter Rühren auf 110 bis 115°C (Temperatur im Reaktionsgemisch), beläßt die Reaktion für 15 Minuten bei dieser Temperatur und kühlt das Reaktionsgemisch auf 20°C. Nach Zugabe von 500 ml Diisopropylether und 500 ml 1% NaCl wird die obere Diisopropyletherphase abgenommen und das Lösungsmittel im Vakuum entfernt (DC-Kontrolle - Rf-Werte in Hexan/Diisopropylether (1:1): Edukt, Rf = 0.2; Produkt, Rf = 0.4).

### Abspaltung der Propenyl-Schutzgruppe

Der Rückstand aus obiger Reaktion, ca. 168 g, wird in 500 ml Methanol gelöst und unter Rückfluß nach Zugabe von 50 ml 1 M HCl gekocht. Nach 60 Minuten ist die Reaktion beendet (DC-Kontrolle in Hexan/Diisopropylether (1:1): Edukt, Rf = 0.4; Produkt, Rf = 0). Die Ausbeute an rac-G₁-3.1-rac-G₂-2-0-benzylether beträgt > 90%. Unter den sauren Bedingungen der Propenylabspaltung wird auch die Isopropylidenschutzgruppe entfernt. Bei Bedarf kann die Isopropylidenschutzgruppe wieder in die 1.2-Position eingeführt werden.

### Einführung der Isopropyliden-Schutzgruppe

Der Rückstand der obigen Reaktion (ca. 0.5 Mol) wird in 300 ml THF gelöst, nacheinander mit 2.2-Dimethoxypropan (0.5 Mol - 52 g) und 0.2 g H₂SO₄ in 10 ml THF versetzt und 2 Stunden bei 25°C gerührt. Das Reaktionsgemisch wird mit gesättigter Na₂CO₃-Lösung neutralisiert, der Niederschlag abgesaugt und das Filtrag im Vakuum mit Xylol einrotiert und dadurch von Wasser befreit. Das Produkt wird durch Chromatographie an Kieselgel 60 (Merck, Korngröße 0.2 - 0.5 mm) gereinigt (Rf-Werte in Diethylether: Edukt, Rf = 0.0; Produkt, Rf = 0.4). Man erhält 121 g des für die Herstellung von Phosphatidyl-diglycerinen (G₂-Grundkörper) wichtigen Zwischenproduktes.

### G₂-Grundgerüst: 2-0-Benzyl-rac-G₁-1.3-0.0-1.2-isopropyliden-rac-G₂ (Ausbeute 82%).

| Summenformel: C₁₆H₂₄O₅ (MG 296.36) | | | |
|---|---|---|---|
| ber. | C, 64.85; | H, 8.16; | 0, 26.99 |
| gef. | C, 64.82; | H, 8.14; | 0, - |

Entsprechend können die für die Herstellung der Phosphatidyloligoglycerine wichtigen Zwischenprodukte mit höheren Oligoglycerinanteilen hergestellt werden. Einige Analysedaten für zentrale Zwischenprodukte sind nachfolgend zusammengestellt worden:

### G₃-Grundgerüst: 2-0-Benzyl-rac-G₁-1.3-0.0-2-0-benzyl-rac-G₂-1.3-0.0-1.2-isopropyliden-rac-G₃

| Summenformel: C₂H₃₆O₇ (MG 460.56) | | | |
|---|---|---|---|
| ber. | C, 67,81; | H, 7.88; | 0, 24.32 |
| gef. | C, 67,75; | H, 7.85; | O, - |

### G₄-Grundgerüst:

### 2-0-Benzyl-rac-G₁-[1.3-0.0-2-O-benzyl-G]₂-1.3-0.0-1.2-isopropyliden-rac-G₄

| Summenformel: C₃₆H₄₈O₉ (MG 624.77) | | | |
|---|---|---|---|
| ber. | C, 69.21; | H, 7.74; | 0, 23.05 |
| gef. | C, 69.17; | H, 7.69; | 0, - |

### G₆-Grundgerüst:

### 2-0-Benzyl-rac-G₁[1.3-0.0-2-0-benzyl-rac-G]₄-1.3-0.0-1.2-isopropyliden-rac-G₆

| Summenformel: C₅₆H₇₂O₁₃ (MG 953.172) | | | |
|---|---|---|---|
| ber. | C, 70.57; | H, 7.61; | O, 21.82 |
| gef. | C, 70.56; | H, 7.54; | 0, - |

### G₈-Grundgerüst:

### 2-0-Benzyl-rac-G₁-[1.3-0.0-2-0-benzyl-rac-G]₆-1.3-0.0-1.2-isopropyliden-rac-G₈

| Summenformel: C₇₆H₉₆O₁₇ (MG 1281.58) | | | |
|---|---|---|---|
| ber. | C, 71.23; | H, 7.55; | 0, 21.22 |
| gef. | C, 71,15; | H, 7.53; | 0, - |

### Beispiel 4d:

### Substanzen, die die Tetrahydropyranyl-Schutzgruppe tragen (anstelle von Benzyl)

### (Herstellung von Phosphatidyl-oligoglycerinen, die ungesättigte Fettsäuren enthalten)

Für die Durchführung dieser Variante wird 1.2-Isopropyliden-rac-glycero-3-0-allylether nach H. Eibl und P. Woolley, Chem. Phys. Lipids 41 (1986) 53-63, hergestellt und epoxidiert.

### Epoxidation (allgemeines Beispiel)

1.2-Isopropyliden-rac-glycero-3-0-allylether (MG 172.22; 1 Mol - 172 g) wird in 1 l CH₂Cl₂ gelöst. 3-Chlorperoxybenzoesäure (1.1 Mol) wird portionsweise zugegeben und für 6 Stunden bei 25 - 30°C gerührt. Das Edukt (Rf 0.5 in Diethylether/Pentan 1:1) hat sich vollständig in das gewünschte Produkt (Rf 0.2 in obigem System) umgesetzt. Man saugt vom Niederschlag ab, gibt zum Filtrat 100 g Na₂CO₃ und rührt für weitere 3 Stunden bei 20°C. Man saugt den Niederschlag ab und entfernt das Lösungsmittel im Vakuum. Die Ausbeute an Epoxid (MG 188.22) beträgt 170 g (90%). Das Epoxid wird nun, wie unter Epoxidöffnung mit Alkoholen (Beisp. 4a) beschrieben, mit Benzylalkohol in 1-0-Benzyl-rac-G₁-3.1-0.0-2.3-isopropyliden-rac-G₂ umgewandelt und die freie OH-Gruppe mit 3.4-Dihydro-2H-Pyran in das Tetrahydropyranderivat umgewandelt.

### Einführung der Tetrahydropyran-Schutzgruppe (allgemeines Beispiel)

1-0-Benzyl-rac-G₁-3.1-0.0-2.3-isopropyliden-rac-G₂ (MG 296.36; 1 Mol - 296 g) wird in 1 l THF gelöst, mit 1.4 Mol 3.4-Dihydro-2H-Pyran versetzt und 0.1 Mol Toluolsulfonsäure zugegeben. Nach 1 Stunde ist die Umsetzung abgeschlossen (Edukt, Rf 0.65; Produkt, Rf 0.90 in Diethyiether). Man versetzt mit 1 l 0.2 Mol Na₂CO₃ Lösung und mit 1 l Diisopropylether und schüttelt in einem Scheidetrichter gut durch. Die obere Phase wird einrotiert und das Produkt durch Hydrogenolyse mit H₂ in Gegenwart eines Pd/C-Katalysators (5% Pd auf Alkohole) in den G₂-Baustein mit freier Hydroxylgruppe umgewandelt.

### G2-Grundgerüst: 2-0-Tetrahydropyranyl-rac-G₁-1.3-0.0-1.2-isopropyliden-rac-G₂ (Ausbeute 80% bezogen auf das Epoxid)

| Summenformel: C₁₄H₂₇O₆ (MG 291.36) | | | |
|---|---|---|---|
| ber. | C, 57.71; | H, 9.34; | 0, 32.95 |
| gef. | C, 57.59; | H, 9.29; | 0, - |

Entsprechend können die Verbindungen mit anderen Grundgerüsten in THP-geschützte Strukturen umgewandelt werden. Z.B. kann der 3-O-Allylether von Beispiel 4a in ein Epoxid umgewandelt und mit Allylalkohol geöffnet werden. Es entsteht wieder ein 3-O-Allylether, der epoxidiert und mit Benzylalkohol geöffnet wird zu unten stehendem Produkt, das durch Einführung von 3 THP-Schutzgruppen und katalytische Hydrogenolyse in ein Zwischenprodukt mit G₄-Grundgerüst umgewandelt werden kann.

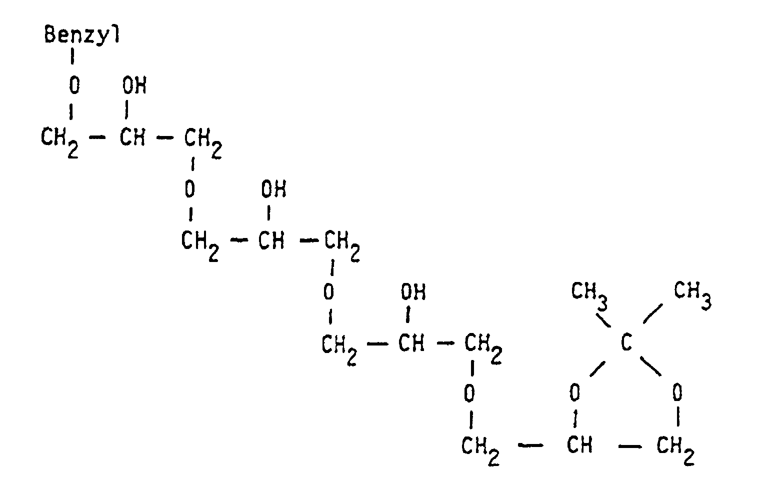

### G₄-Grundgerüst:

### 2-O-THP-rac-G₁[1.3-0.O-2-0-THP-rac-G]₂-1.3-0.0-1.2-isopropyliden-rac-G₄

| Summenformel: C₃₀H₄₅O₂ (MG 607.75) | | | |
|---|---|---|---|
| ber. | C, 59.29; | H, 9.12; | 0, 31.59 |
| gef. | C, 59.24; | H, 9.08; | O, - |

### Beispiel 4e:

### Weiterverarbeitung des Zwischenprodukts der Formel I

### G₂-Grundkörper (racemisch)

Aus Formel I kann ein zentrales Zwischenprodukt für die Herstellung der G₂-Grundkörper erhalten werden (siehe Schema B). Dazu wird die sekundäre OH-Funktion in Formel I alkyliert, benzyliert oder mit Tetrahydropyranyl geschützt.

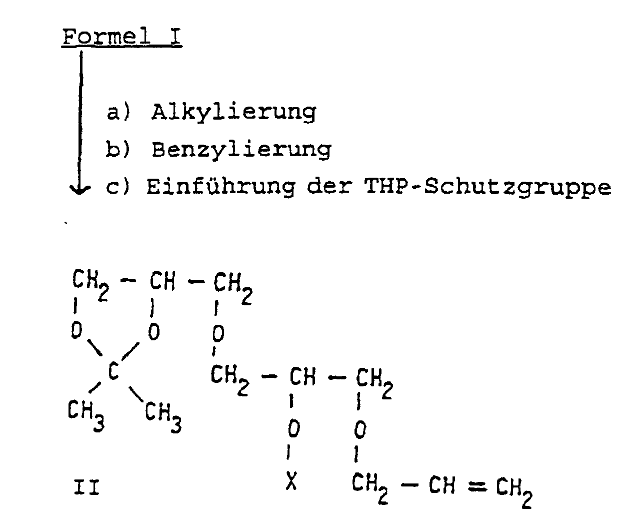

### Schema B: Zentrales Zwischenprodukt zur Herstellung der G₂-Grundkörper:

X = gesättigtes oder ungesättigtes Alkyl, Benzyl oder THP

### Alkyl-G₂-Verbindungen

### 1) 2-0-Alkyl-rac-G₁-1.3-0.0-rac-G₂

Die Zwischenverbindung der Formel II mit X = Alkyl wird von den Schutzgruppen befreit. Es wurden folgende Verbindungen dargestellt:

| | | |
|---|---|---|
| 2-O-Ethyl-G₂ | C₈H₁₈O₅ | (194.23) |
| 2-O-Hexyl-G₂ | C₁₂H₂₆O₅ | (250.33) |
| 2-O-Undecenyl-G₂ | C₁₇H₃₄O₅ | (318.45) |
| 2-O-Dodecyl-G₂ | C₁₈H₃₈O₅ | (334.49) |
| 2-O-Octadecyl-G₂ | C₂₄H₅₀O₅ | (418.65) |
| 2-O-Erucyl-G₂ | C₂₈H₅₆O₅ | (472.75) |

### 2) 1-O-Alkyl-rac-G₁-3.1-0.0-rac-G₂

In der Zwischenverbindung der Formel II mit X = Benzyl wird Allyl aus der 1-Position entfernt und die entsprechende Alkylkette in 1-Position eingeführt. Nach Entfernung der Schutzgruppen wurden folgende Verbindungen erhalten:

| | | |
|---|---|---|
| 1-O-Methyl-G₂ | C₇H₁₆O₅ | (180.20) |
| 1-O-Propyl-G₂ | C₉H₂₀O₅ | (208.25) |
| 1-O-Nonyl-G₂ | C₁₅H₃₂O₅ | (292.41) |
| 1-O-Undecyl-G₂ | C₁₇H₃₆O₅ | (320.47) |
| 1-O-Dodecyl-G₂ | C₁₈H₃₈O₅ | (334.49) |
| 1-O-Octadecyl-G₂ | C₂₄H₅₀O₅ | (418.65) |

Ungesättigte 1-O-Alkyldiglyceride können auch direkt über Epoxidöffnung von 1.2-Isopropyliden-glycero-glycidylether (Schema D, Formel IV) mit Alkoholen erhalten, z.B.

| | | |
|---|---|---|
| 1-O-Undecenyl-G₂ | C₁₇H₃₄O₅ | (318.45) |

Dieser Weg ist jedoch nur für kürzerkettige Alkohole geeignet, da die Ausbeuten für längerkettige Alkohole wie z.B. Oleylalkohol gering sind. Zur Herstellung von 1-Oleyl-G₂ wird deshalb ein Syntheseweg über 2-O-THP-glycero-1.3-0.0-(1.2-isopropyliden)-glycerin vorgezogen (Schema D, Formel V)

| | | |
|---|---|---|
| 1-O-Oleyl-G₂ | C₂₄H₄₈O₅ | (416.64) |

### Zwischenprodukte für die Synthese von Phospholipiden, die Dicylcerine im polaren Bereich enthalten

Verbindungen mit günstigen Schutzgruppen für diese Synthesen enthalten in G₁ eine 2-O-Benzylether- oder eine 2-O-Tetrahydropyranylethergruppe:

| | | | |
|---|---|---|---|
| 1) | 2-O-Benyzl-rac-G₁-1.3-0.0-(1.2-isopropyliden)-rac-G₂ | C₁₆H₂₄O₅ | (296.36) |

Die Verbindung der Formel III wird durch basische Umlagerung von Allyl in Propenyl, Benzylierung der sekundären OH-Gruppe und anschließende saure Abspaltung der Propenylschutzgruppe erhalten.

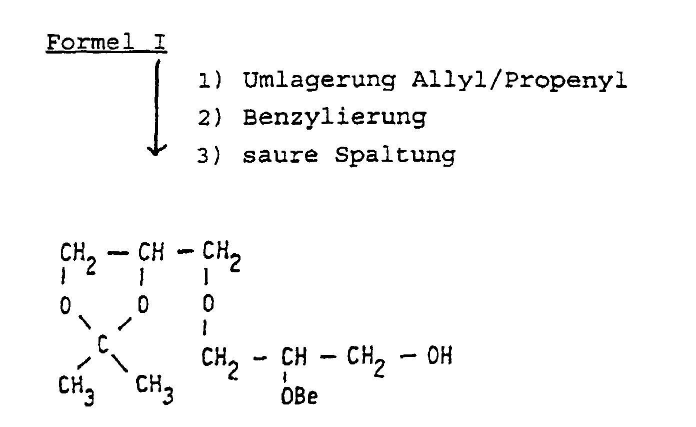

### Schema C: Ausganasprodukt für Phosphatidyl-di-glycerine mit gesättigten Fettsäureresten.

| | | | |
|---|---|---|---|
| 2) | 2-O-Tetrahydropyranyl-rac-G₂-1.3-0.0-(1.2-isopropyliden) - rac-G₂ | C₁₄G₂₇O₆ | (291.36) |

Die Verbindung der Formel V wird aus Allylglycerin hergestellt. Nach Anlagerung von Isopropyliden wird epoxydiert und man erhält das Zwischenprodukt IV. nach Öffnung des Epoxids mit Benzylalkohol wird die THP-Schutzgruppe eingeführt und die Benzylgruppe entfernt.

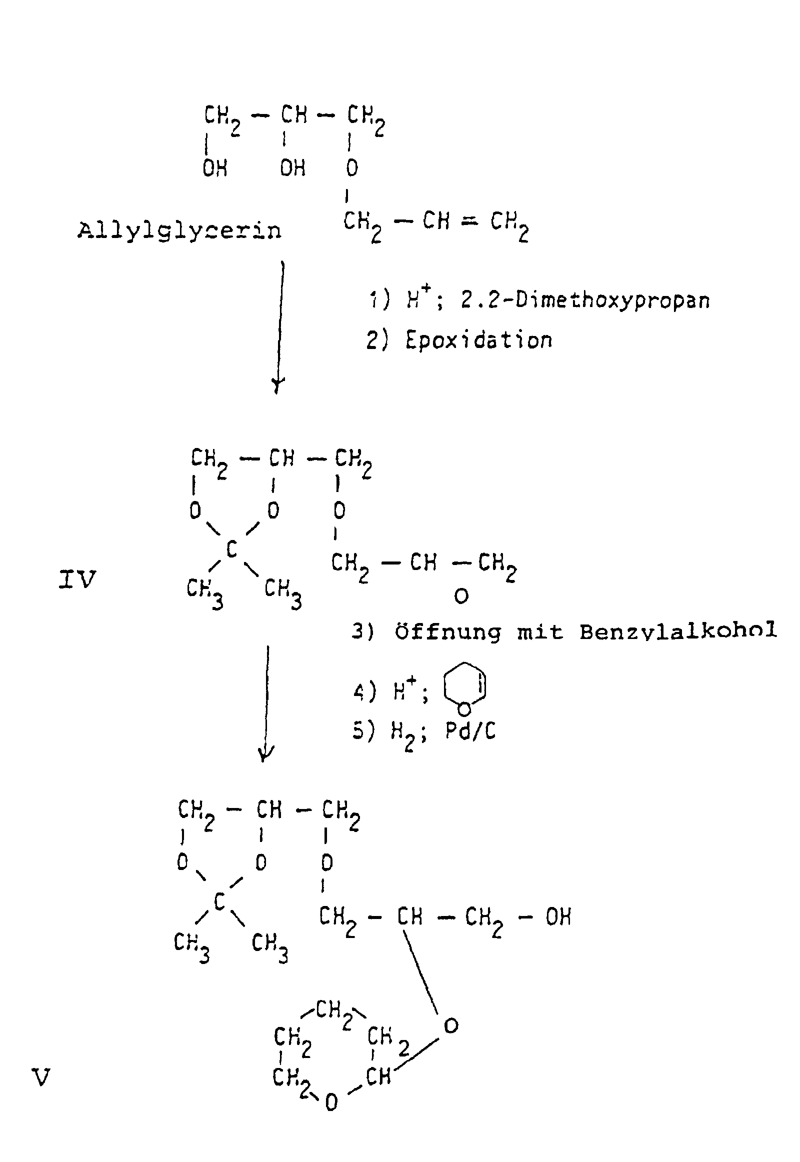

### Schema D: Ausgangsprodukt für Phosphatidyl-di-glycerine mit ungesättigten Fettsäureresten

### G₃-Grundkörper (racemisch)

Aus dem zentralen Zwischenprodukt der Formel II können unter Einbeziehung der Allylgruppe die Triglyceride entwickelt werden. Nach Epoxidation können aus dem Epoxid verschiedene Zwischen- oder Endprodukte von pharmazeutischen Interesse hergestellt werden.

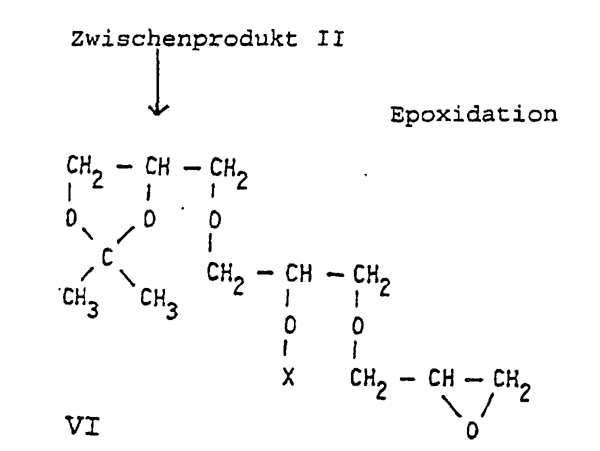

### Schema E: Ausgangsprodukte für die Herstellung von G₃-Grundkörpern.

Aus dem zentralen Zwischenprodukt der Formel VI können Triglycerine hergestellt werden; das Zwischenprodukt dient auch zur Herstellung des G₄-Grundkörpers. In der Formel VI stellt X Wasserstoff, einen gesättigten Alkyl-, einen Benzyl- oder einen THP-Rest dar.

### Alkyl-G₃-Verbindungen

### 1) 1-O-Alkyl-rac-G₁-1.3-0.0-rac-G₂-1.3-0.0-rac-G₃

Das Epoxid der Formel VI (X = H) wird mit Alkoholen direkt geöffnet und ergibt nach Abspaltung der Isopropyliden-Schutzgruppe folgende Verbindungen:

| | | |
|---|---|---|
| 1-O-Ethyl-G₃ | C₁₁H₂₄O₇ | (268.30) |
| 1-O-Hexyl-G₃ | C₁₅H₃₂O₇ | (324.41) |
| 1-O-Nonyl-G₃ | C₁₈H₃₈O₇ | (366.491) |
| 1-O-Undecenyl-G₃ | C₂₀H₄₀O₇ | (392.53) |
| 1-O-Dodecyl-G₃ | C₂₁H₄₄O₇ | (408.57) |

Für längerkettige Alkohole sind die Ausbeuten bei der direkten Öffnung schlecht. Deshalb wurden die Oleyl- und Erucyl-Verbindungen von G₃ durch Öffnung von VI (X = THP) mit Benyzl, Schutz der gebildeten sekundären Hydroxylgruppen mit THP, katalytische Debenzylierung, Alkylierung in 1-Position und Entfernung der Schutzgruppen hergestellt.

| | | |
|---|---|---|
| 1-O-Oleyl-G₃ | C₂₇H₅₄O₇ | (490.72) |
| 1-O-Erucyl-G₃ | C₃₁H₆₂O₇ | (456.82) |

### 2) 2-O-Alkyl-rac-G₁-1.3-0.0-rac-G₂-1.3-0.0-rac-G₃

Das Epoxid der Formel VI (X = Benzyl oder THP) wird mit Allyl-alkohol geöffnet und in der 2-Position alkyliert. Die Schutzgruppen werden auf übliche Weise entfernt. Bei der Herstellung der ungesättigten 2-0-Alkylverbindungen muß die Umlagerung der Allyl-Schutzgruppe vor der Alkylierung erfolgen. Außerdem kann in diesem Fall in G₂ nur die THP-Schutzgruppe, nicht aber Benzyl verwendet werden. Folgende Verbindungen wurden hergestellt:

| | | |
|---|---|---|
| 2-O-Methyl-G₃ | C₁₀H₂₂O₇ | (111.99) |
| 2-O-Propyl-G₃ | C₁₂H₂₆O₇ | (282.33) |
| 2-O-Nonyl-G₃ | C₁₈H₃₈O₇ | (366.49) |
| 2-O-Undecenyl-G₃ | C₂₀H₄₀O₇ | (392.53) |
| 2-O-Dodecyl-G₃ | C₂₁H₄₄O₇ | (408.57) |
| 2-O-Hexadecyl-G₃ | C₂₅H₅₂O₇ | (464.68) |
| 2-O-Oleyl-G₃ | C₂₇H₅₄O₇ | (490.72) |
| 2-O-Erucyl-G₃ | C₃₁H₆₂O₇ | (546.82) |

### Zwischenprodukte für die Synthese von Phospholipiden, die Triglyceride im polaren Bereich enthalten

Günstige Schutzgruppen für den Aufbau von Phospholipiden, die G₃-Reste im polaren Bereich enthalten, sind Benzylreste und Tetrahydropyranylreste (THP). Benzylreste können einfach und unter milden Bedingungen entfernt werden, allerdings mit der Einschränkung, daß nur gesättigte Fettsäuren verwendet werden können. THP-Reste sind von besonderem Interesse, da sie in Verbindung mit Isopropylschutzgruppen in einem Schritt abgelöst werden können.

| | | | |
|---|---|---|---|
| 1) | 2-O-Benyzl-rac-G₁-1.3-0.0-(2-O-Benzyl)-rac-G₂-1.3-0.0-(1.2-isopropyliden)-rac-G₃ | C₂₆H₃₆O₇ | (460.56) |

Diese Verbindung kann aus dem zentralen Zwischenprodukt VI (X = Benzyl) durch Öffnen mit Allylalkohol, Benzylierung der 2-Position und Abspaltung der Allylschutzgruppe erhalten werden. Sie wird im Text als Formel VII bezeichnet.

| | | | |
|---|---|---|---|
| 2) | 2-O-THP-rac-G₁-1.3-0.0-(2-O-THP)-rac-G₂-1.3-0.0-(1.2-iso -propyliden)-rac-G₃ | C₂₂H₄₁O₉ | (449.56) |

Zur Darstellung ungesättigter G₃-Phospholipide wird in VI der Rest X = THP verwendet. Man öffnet das Epoxid VI mit Benzylalkohol, schützt die freiwerdende sekundäre Hydroxylgruppe mit THP und entfernt den Benzylrest katalytisch mit H₂/Pt. Die so hergestellte Verbindung wird im Text als Formel VIII bezeichnet.

### Zusätzliche Bemerkungen

In der bisherigen Beschreibung haben wir von der Tatsache nicht Gebrauch gemacht, daß in der Formel VI für X = gesättigtes Alkyl einfach Verbindungen folgender Struktur hergestellt werden können: 1-O-Alkyl-rac-G₁-3.1-0.0-(2-0-alkyl)-rac-G₂-3.1-rac-G₃. Die Vertreter dieser neuen Strukturen wurden hergestellt durch Öffnen des Epoxids VI (X = Hexadecyl) mit CH₃OH oder Undecenylalkohol und Abspaltung der Isopropyliden Schutzgruppe

| | | |
|---|---|---|
| 1-O-Methyl-rac-G₁-3.1-O.O-(2.O-hexadecyl)-rac-G₂-3.1-rac-G₃ | C₂₆H₅₄O₇ | (478.71) |

| | | |
|---|---|---|
| 1-O-Undecenyl-rac-G₁-3.1-O.O-(2-O-hexadecyl)-rac-G₂-3.1-O.O-rac-G₃ | C₃₆H₇₂O₇ | (616.958) |

### G₄-Grundkörper (racemisch)

Aus dem zentralen Zwischenprodukt der Formel IX können G₄-Grundkörper hergestellt werden.

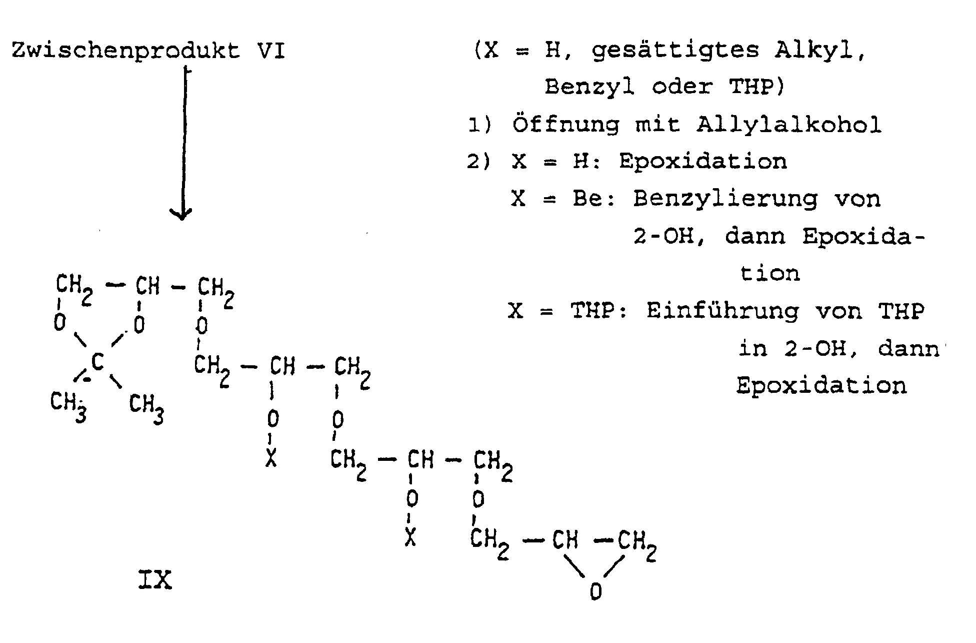

### Schema F: Ausgangsprodukte für die Herstellung von G₄-Grundkörpern.

Aus dem zentralen Zwischenprodukt der Formel IX können Tetraglycerine hergestellt werden. Sie können auch zur Herstellung von Pentaglycerinen verwendet werden.

Aus den Zwischenverbindungen können auch Oligoglycerine mit zwei oder mehr Alkylresten hergestellt werden. Geeignete Ausgangsverbindungen hierfür sind Moleküle der Formel IX in denen X einen gesättigten Alkylrest darstellt.

### Alkyl-G₄-Verbindungen

### 1) 1-O-Alkyl-rac-G₁-1.3-0.0-rac-G₂-1.3-0.0-rac-G₃-1.3-0.0-rac-G₄

Das Epoxid der Formel IX, X = H, wird mit Alkoholen direkt geöffnet. Das ergibt nach Abspaltung der Isopropyliden-Schutzgruppe folgende Substanzen:

| | | |
|---|---|---|
| 1-0-Ethyl-G₄ | C₁₄H₃₀O₉ | (342.38) |
| 1-0-Hexyl-G₄ | C₁₈H₃₈O₉ | (398.49) |
| 1-0-Undecyl-G₄ | C₁₉H₄₀O₉ | (412.52) |
| 1-0-Undecenyl-G₄ | C₁₉H₃₈O₉ | (410.50) |
| 1-0-Dodecyl-G₄ | C₂₀H₄₂O₉ | (426.54) |

Dieser Weg ist nur für kürzerkettige Alkohole geeignet, da die Ausbeuten für längerkettige Alkohole stark abfallen.

Für längerkettige Alkohole, die gesättigt sind, muß deshalb wie bei G₂ und G₃ ein Syntheseweg über das zentrale Zwischenprodut mit X = Benzyl gewählt werden. Man öffnet mit Allylalkohol, benzyliert die freiwerdende 2-OH-Gruppe, entfernt die Allylgruppe in der 1-Position und alkyliert die 1-Position. Nach Entfernen der Schutzgruppen erhält man:

| | | |
|---|---|---|
| 1-0-Hexadeyl-G₄ | C₂₄H₅₀O₉ | (482.99) |
| 1-0-Octadecyl-G₄ | C₂₆H₅₄O₉ | (510.70) |
| 1-0-Behenyl-G₄ | C₃₀H₆₂O₉ | (566.81) |

### 2. 2-O-Alkyl-rac-G₁-1.3.-0.0-rac-G₂-1.3-0.0-rac-G₃-1.2-0.0-rac-G₄

Die zentrale Zwischenverbindung der Formel IX wird mit Allylalkohol geöffnet und die freiwerdende 2-Position alkyliert. Nach Entfernen der Schutzgruppen erhält man:

| | | |
|---|---|---|
| 2-0-Propyl-G₄ | C₅H₃₂O₉ | (356.41) |
| 2-0-Hexyl-G₄ | C₁₈H₃₈O₉ | (398,49) |
| 2-0-Nonyl-G₄ | C₂₁H₄₄O₉ | (440.57) |
| 2-0-Undenyl-G₄ | C₁₉H₃₈O₉ | (410.50) |
| 2-O-Dodecyl-G₄ | C₂₀H₄₂O₉ | (426.54) |
| 2-0-Hexadecyl-G₄ | C₂₄H₄₀O₉ | (483.99) |
| 2-0-Octadecyl-G₄ | C₂₆H₅₄O₉ | (510.70) |
| 2-0-Oleyl-G₄ | C₂₆H₅₂O₉ | (508.69) |
| 2-0-Erucyl-G₄ | C₃₀H₆₀O₉ | (564.80) |

### Zwischenprodukte für die Synthese von Phospholipiden, die Tetraglycerine im polaren Bereich enthalten.

Verbindungen mit günstigen Schutzgruppen für diese Synthesen sind entsprechend den Erfahrungen für die Synthese von G₂- und G₃-Verbindungen Benzyl- und Tetrahydropyranylether.

| | | | |
|---|---|---|---|
| 1) | 2-O-Benzyl-rac-G₁-1.3-0.0-(2-0-benzyl)-rac-G₂-1.3-0.0-(2-0-benzyl)-rac-G₃-1.3-0.0-(1.2-isopropyliden)-rac-G₄ | C₃₆H₄₈O₉ | (624.77) |

Die zur Synthese von Phospholipiden mit G₄-Resten im polaren Bereich wichtige Zwischenverbindung wird aus Formel IX, X = Benzyl durch Öffnen des Epoxids mit Allylalkohol, anschließender Benzylierung der freiwerdenden 2-OH-Gruppe und Entfernung von Allyl hergestellt. Die Verbindung erscheint im Text als Formel X.

| | | | |
|---|---|---|---|
| 2. | 2-0-THP-rac-G₁-1.3-0.0-(2-0-THP)-rac-G₂-1.3-0.0-(2-0-THP)-rac-G₃-1.3-0.0-(1.2-isopropyliden)-rac-G₄ | C₃₀H₄₅O₂ | (607.75) |

Zur Herstellung dieser Verbindung, die zur Darstellung ungesättigter Phospholipide mit G₄-Grundkörpern im polaren Bereich geeignet ist, geht man analog vor, wie bei der Herstellung der G₃-Verbindung. Man öffnet das Epoxid VII, X = THP mit Benzylalkohol, schützt die freiwerdende 2-OH-Gruppe mit THP und entfernt Benzyl mit H₂ (Pd/C-Katalyse). Die Verbindung wird im Text unter Formel XI genannt.

### ZWISCHENPRODUKTE FÜR DIE SYNTHESE VON PHOSPHOLIPIDEN, DIE OLIGOGLYCERINE IM POLAREN BEREICH ENTHALTEN UND EINE SN-1-VERKNÜPFUNG ZUM PHOSPHAT ERMÖGLICHEN (NATÜRLICHE KONFIGURATION)

Bei der bisherigen Herstellung der Verbindungen, die für einen Einbau in den polaren Bereich von Phospholipiden geeignet sind (Formeln III und V für G₂, Formeln VII und VIII für G₃, Formeln X und XI für G₄), wurde nicht darauf geachtet, daß in natürlichem Phosphatidylglycerin, d.h. in 1.2-Diacyl-sn-glycero-3-phospho-sn-1-glycerin, die Verknüpfung von Phosphat mit dem nicht acylierten Glycerin eine sn-1-Verknüpfung ist. Da die Liposomenbestandteile als Karrier von Arzneimitteln möglichst in natürlicher Konfiguration verwendet werden, wurden Synthesewege entwickelt, die auch eine sn-1-Konfiguration des polaren Oligoglycerins zulassen (Schema G).

### sn-1-G₁-G₂-Verknüpfung

Die stereospezifische Verknüpfung kann analog zu den in der Literatur beschriebenen Verfahren durchgeführt werden (DE 31 30 867 A1; H. Eibl, Chem. Phys. Lipids 28 (1981) 1-5; H. Eibl et al., Chem. Phys. Lipids 41 (1986) 53-63; H. Eibl et al., Chem. Phys. Lipids 47 (1988) 47-53).

Das Ausgangsprodukt für diese Verknüpfung ist 2-0-Benzyl-3-0-allyl-sn-glycerin, das nach Epoxidation durch Hydrolyse in das Diol umgewandelt wird. Nach Umsetzung mit H⁺/2.2-Dimethoxypropan entsteht 2-0-Be-sn-G₁-3.1-0.0-(1.2-isopropyliden)-rac-G₂, ein Molekül der Formel XII, das eine sn-1-Verknüpfung zur Phosphatgruppe in Phospholipiden ermöglicht und dem Racemat der Formel III entspricht.

### sn-1-G₁-G₂-G₃-Verknüpfung

Das Ausgangsprodukt für diese Verknüpfung ist wieder 2-0-Benzyl-3-0-allyl-sn-glycerin. Nach Schutz der sn-1-Position mit THP wird epoxidiert und der Epoxidring mit 1.2-Isopropylidenglycerin geöffnet. Man benzyliert die frei werdende OH-Punktion in G₂, entfernt die THP-Schutzgruppe und erhält das Molekül der Formel XIII, das eine sn-1-Verknüpfung zur Phosphatgruppe in Phospholipiden ermöglicht. Das Molekül XIII entspricht dem Racemat der Formel VII.

### sn-1-G₁-G₂-G₃-G₄-Verknüpfung

Ausgangsprodukt ist wieder 2-0-Benzyl-3-0-allyl-sn-glycerin, um die sn-1-Verknüpfung zu gewährleisten. Nach Einführung der THP-Schutzgruppe wird epoxidiert und das Epoxid mit Allylalkohol geöffnet. Nach Epoxidation des Zwischenproduktes wird mit Isopropylidenglycerin geöffnet, die beiden freien OH-Gruppen benzyliert und THP entfernt. Man erhält XIV, das eine sn-1-Verknüpfung zum Phosphat ermöglicht und dem Racemat der Formel X entspricht.

Wenn erwünscht, können entsprechend auch Verbindungen mit sn-3-G₁-G₂-, sn-3-G₁-G₂-G₃- oder sn-3-G₁-G₂-G₃-G₄-Verknüpfung zum Phosphat hergestellt werden. In diesem Falle muß in einer, gleichen Sequenz von Reaktionen anstelle von 2-0-Benzyl-2-0-allyl-sn-glycerin das enantiomere 2-0-Benzyl-1-0-allyl-snglycerin eingesetzt werden.

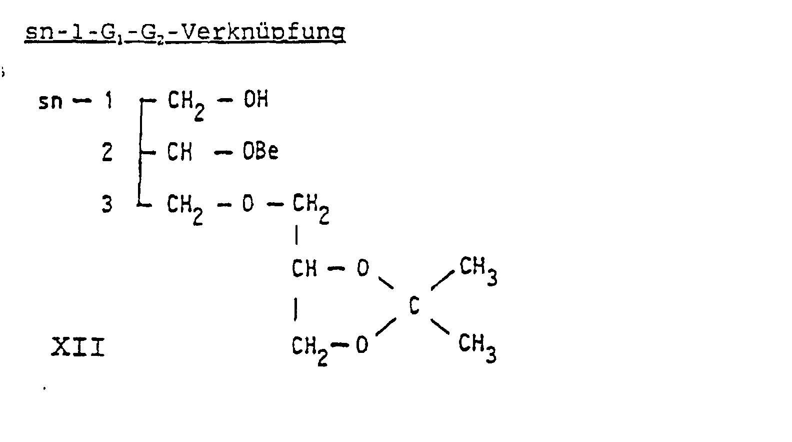

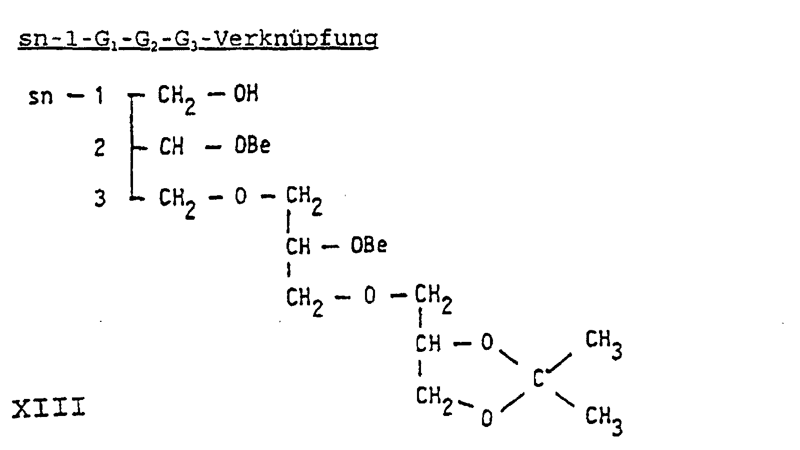

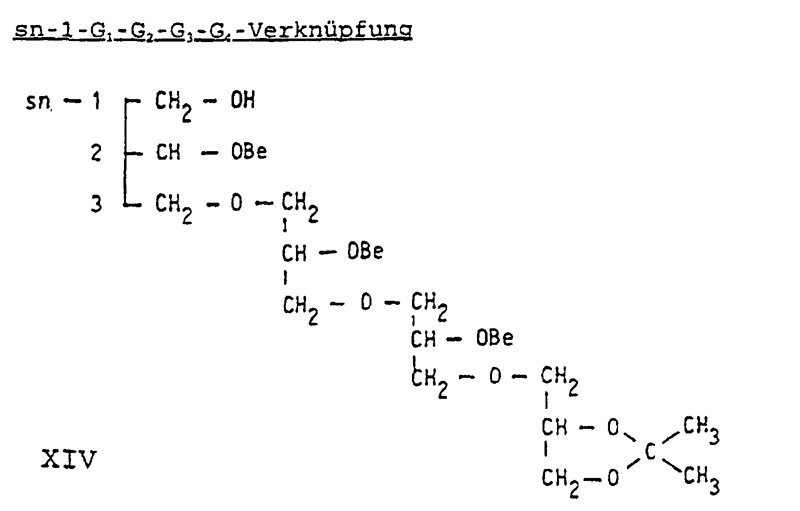

### Schema G: Bausteine für Phospholipide, die eine sn-1-Gₓ-Verknüpfung ermöglichen (x = 2 - 4). Ausgangsprodukt ist 2-O-Benzyl-3-O-allyl-sn-glycerin.

### Beispiel 4f:

### Zwischenprodukte, die Zuckeralkohole enthalten (allgemeine Beispiele)

Wichtige Zwischenprodukte sind hier vor allem solche Zuckeralkohole, die preislich günstig zu erwerben oder aus diesen durch einfache Umsetzungen erreicht werden können (siehe dazu beiliegende Tabelle). Interessant sind insbesondere D-Mannit als offene Form von Inosit, -Xylit, das bei einer Phsophorylierung am mittleren C-Atom keine optische Aktivität besitzt und das als 1.2; 4.5-Diisopropyliden-Xylit leicht zu erhalten ist, sowie meso-Erythrit. Als Schutzgruppen werden hier meistens Isopropyliden, Trityl in Kombination mit Benzyl oder Allyl eingesetzt. Auch die Tetrahydropyranyl-Schutzgruppe hat hier eine gewisse Bedeutung. Beispielhaft werden hier einige Möglichkeiten beschrieben.

### 1.2;4.5-Diisopropyliden-Xylit (allgemeines Beispiel für die Einführung der Isopropyliden-Schutzgruppe)

Xylit (1.0 Mol - 152 g) wird in 500 ml 2-Propanol aufgeschlämmt und Dimethoxypropan (3.0 Mol - 312 g) zugegeben. Man versetzt mit 6 g H₂SO₄ in 100 ml 2-Propanol und erwärmt auf 50 °C. Nach 30 Minuten ist alles gelöst. Man versetzt mit soviel konzentriertem Ammoniak, bis das Reaktionsgemisch einen pH-Wert von ungefähr 8 aufweist. Nach Entfernen des Lösungsmittels am Rotationsverdampfer in Vakuum wird der Rückstand in Hexan aufgenommen und auf -20 °C gekühlt. Es fallen schöne, weiße Kristalle aus, die abgesaugt und für die Phosphorylierung verwendet werden.

| Summenformel: C₁₁H₁₉O₅ (MG 231.27) | | | |
|---|---|---|---|
| ber. | C, 57.13; | H, 8.28; | 0, 34.59 |
| gef. | C, 57.01; | H, 8,27; | 0, - |

Strukturformeln einiger Zuckeralkohole:

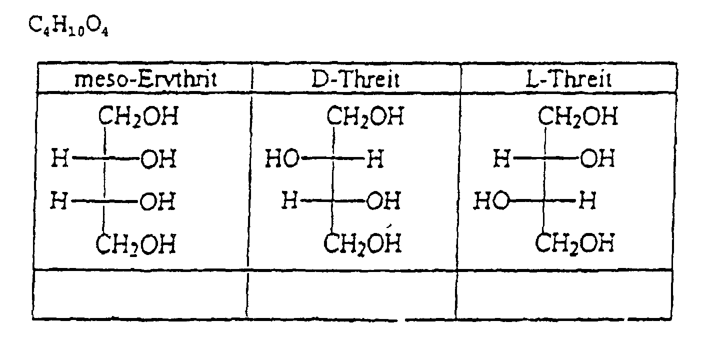

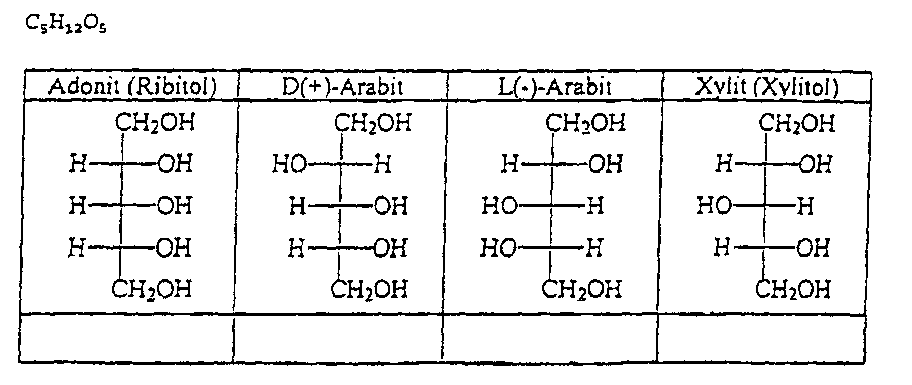

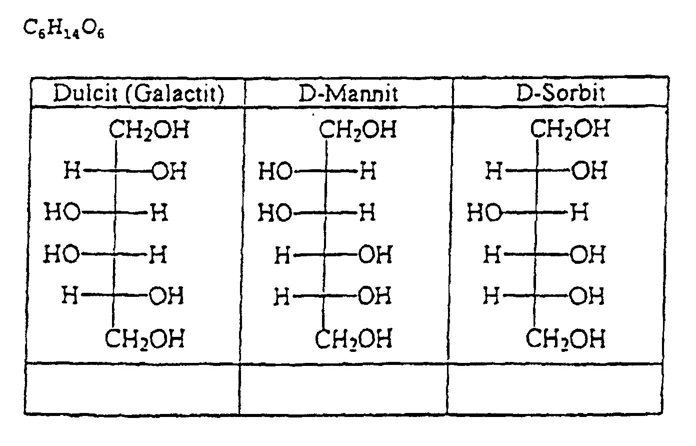

### 1.2;3.4-Diisopropyliden-5-benzyl-D-Mannit (allgemeines Beispiel für die Verwendung von Tritylschutzgruppen in Verbindung mit Benzylschutzgruppen)

Ausgehend von 1.2;3.4;5'.6-Triisopropyliden-D-Mannit (MG 302.36), das analog zur Herstellung des Xylit-Derivates erhalten wurde, wird durch vorsichtige Abspaltung der Schutzgruppe in etwa 30 %-iger Ausbeute 1.2.3.4-Diisopropyliden-D-Mannit erhalten. Triisopropyliden-D-Mannit (1.0 Mol - 302 g) wird in 600 ml CH₃OH gelöst, mit Amberlyst 15 (5 g) und 70 g H₂O versetzt. Man erwärmt auf 50 °C und rührt die Lösung bei dieser Temperatur für 40 Minuten (Edukt, RF 0.9; 1.2;3.4-Derivat, Rf 0.7; 3.4-Derivat, Rf 0.1 in CHCl₃/CH₃OH 1:1), kühlt auf 20 °C und filtriert zu 7,5 ml 25 % Ammoniak in 25 ml 2-Propanol (pH - 8). Beim Abkühlen auf 4 °C fällt das Ausgangsprodukt aus und kann zurückgewonnen werden (ca. 120 g, ∼ 40 %). Das Filtrat wird einrotiert und durch Chromatographie an Kieselgel 60 (Merck, Darmstadt) gereinigt. Man erhält 84 g (- 32 %) an 1.2:3,4-Diisopropyliden-D-Mannit, das aus Hexan kristallin erhalten werden kann.

| Summenformel: C₁₂H₂₂O₆ (MG 262.30) | | | |
|---|---|---|---|
| ber. | C, 54.95; | H, 8.45; | O, 36.60 |
| gef. | C, 54.89; | H, 8.34; | 0. - |

### Umsetzung von 1.2;3.4-Diisopropyliden-D-Mannit mit Tritylchlorid und Benzylchlorid (allgemeines Beispiel für die Tritylierunq und anschließende Alkylierung)

1.2:3.4-Diisopropyliden-D-Mannit (0.2 Mol - 52 g) wird in 300 ml Toluol gelöst, mit Triethylamin (0,30 Mol - 30 g) versetzt und unter Rückfluß gekocht. Man tropft Tritylchlorid (MG 278.78; Mol - 64 g) in 200 ml Toluol zu und kocht weitere 60 Minuten unter Rückfluß (Edukt, Rf 0.7; Produkt, Rf 0.90 in CHCl₃/CH₃OH 10:1). Die Reaktion ist dann abgeschlossen. Man kühlt auf 20 °C, filtriert ausgefallenes Triethylaminhydrochlorid ab und rotiert das Filtrat ein. Der obige Rückstand wird in 400 ml THF aufgenommen, mit Benzylchlorid (0.3 Mol - 38 g) versetzt und unter Rückfluß gekocht. Man tropft K-tert.-Butylat (0.25 Mol - 28 g) - gelöst in 200 ml THF - ein und arbeitet das Reaktionsgemisch nach 1 Stunde auf (Edukt, Rf 0.90; Produkt, Rf 1.00 in CHCl₃/CH₃OH 10:1). Man extrahiert das Reaktionsgemisch nach Zusatz von 300 ml Diisopropylether mit 600 ml H₂O, nimmt die obere Phase ab und entfernt das Lösungsmittel im Vakuum. Der obige Rückstand wird direkt weiterverwendet.

### Abspaltung der Tritylschutzgruppe unter Erhalt der 3.4-Isopropyliden-Schutzgruppe (allgemeines Beispiel)

Der ölige Rückstand der vorhergehenden Reaktion (∼0.2 Mol) wird in 600 ml Aceton/CH₃OH 1:1 gelöst und mit 3 ml H₂SO₄ versetzt. Man rührt bei 40 °C für 40 Minuten und beobachtet eine vollständige Abspaltung der Trityl- und der 1-2-Isopropyliden-Schutzgruppe (Edukt, Rf 0.95; Produkt, Rf 0.15 in Ether). Das Reaktionsgemisch wird mit Ammoniak auf pH - 8 gebracht, filtriert und einrotiert. Der Rückstand wird in Kieselgel chromatographiert und aus Hexan kristallisiert.

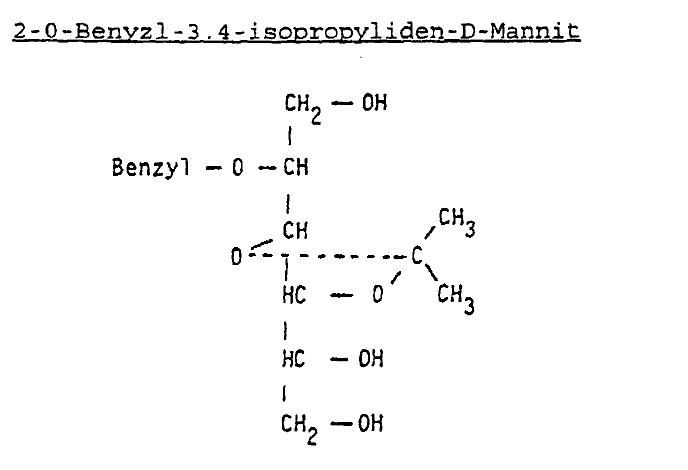

| Summenformel: C₁₆H₂₄O₆ (MG 312.36) | | | |
|---|---|---|---|
| ber. | C, 61.52; | H, 7.74; | 0, 30.73 |
| gef. | C, 61.44; | H, 7.72; | 0, - |

Wie in Beispiel 4c angegeben, kann die Isopropyliden-Schutzgruppe in 5.6-Position wieder eingeführt werden. Es entsteht ein zentrales Zwischenprodukt für die Synthese von Phosphatidyl-D-Mannit-Verbindungen, 2-0-Benzyl-3.4;5.6-diisopropyliden-D-Mannit.

| Summenformel: C₁₉H₂₈O₆ (MG 352.42) | | | |
|---|---|---|---|
| ber. | C, 64.75; | H, 8.01; | 0, 27.24 |
| gef. | C, 64,68; | H, 7.94; | 0, - |

### Bausteine aus Zuckeralkoholen, die durch Kombination einer Perjodatabspaltung eines vicinalen Diols und Reduktion des entstehenden Aldehyds mit Natriumborhydrid erhalten werden (allgemeines Beispiel)

1.2:3.4-Diisopropyliden-D-Mannit (0.2 Mol - 26 g) wird nach H. Eibl, Chem. Phys. Lipids 28 (1981) 1-5, in 200 ml CH₃OH gelöst und zu einer Lösung von 0.2 Mol Natriummetaperjodat in 500 ml Wasser gegeben. Die Temperatur soll 30 °C nicht übersteigen. Nach 15 Minuten ist die Reaktion beendet. Der pH-Wert des Reaktionsgemisches wird mit 5 M KOH in Wasser auf pH = 8 erhöht. Die ausgefallenen Salze werden abfiltriert und der entstandene Aldehyd mit Natriumborhydrid (0,25 Mol) reduziert. Man erhält in > 90 %-iger Ausbeute 1.2:3.4-Diisopropyliden-D-Lyxit, das mit 600 ml Chloroform extrahiert wird. Die Chloro- form-Phase wird einrotiert und das Produkt aus Hexan kristallisiert.

| Summenformel: C₁₁H₁₉O₅ (MG 231.27) | | | |
|---|---|---|---|
| ber. | C, 57.13; | H, 8.28; | 0, 34.59 |
| gef. | C, 57.07; | H, 8.21, | O, - |

Durch Verwendung dieser unterschiedlichen Möglichkeiten, Monoisopropylidenabspaltung, Perjodatabspaltung der vicinalen Diole zu den Aldehyden und Natriumborhydridreduktion in Kombination mit der Variation Trityl/Alkyl können die unterschiedlichsten geschützten Zuckeralkohole erhalten werden, die durch Acylierung oder Phosphorylierung in interessante Alkyl-, Acyl- oder Phosphatidylverbindungen umgewandelt werden können.

### Herstellung einfacher Ester- und Etherderivate aus den geschilderten Oligoglycerinen und Zuckeralkoholen (allgemeine Beschreibung)

Möglichkeiten der Veresterung oder Veretherung mit anschließender Abspaltung der Schutzgruppen wurden in verschiedenen Publikationen beschrieben. In den im folgenden aufgeführten Artikeln finden sich zudem unterschiedliche Methoden der Phosphorylierung. Diese Methoden können hier analog verwendet werden.
Eibl, H.
   Synthesis of glycerophospholipids
   Chem. Phys. Lipids 26 (1980) 405-429
Eibl, H.
   Phospholipid Synthesis
   In: Liposomes: From Physical Structure to Therapeutic Applications (C.G. Knight, editor) Elsevier, Amsterdam (1981) 19-50
Eibl, H. and Kovatchev, S.
   Preparation of phospholipids and of their analogs by phospholipase D. In: Methods of Enzymology. Vol. 72. Ed. J.M. Lowenstein, Academic Press, New York (1981) 632-639
Eibl, H.: Phospholipide als funktionelle Bausteine biologischer Membranen Angew. Chemie 96 (1984) 247-262
Eibl, H.: Phospholipids as functional constituents of biomembranes Angew. Chem. Int. Ed. Engl. 23 (1984) 257-271
Eibl, H. Phospholipid synthesis: Oxazaphospholanes and dioxaphospholanes as intermediates. Proc. Natl. Acad. Sci. USA 75 (1978) 4074-4077
Eibl, H. and Wooley, P.: Synthesis of enantiomerically pure glyceryl esters and ethers. I. Methods employing the precursor 1,2-isorpropylidene-sn-glycerol. Chem. Phys. Lipids 41 (1986) 53-63
Eibl. H. and Wooley, P.: Synthesis of enantiomerically pure glyceryl esters and ethers. II. Methods employing the precursor 3,4-isopropylindene-D-mannitol. Chem. Phys. Kipids 47 (1988) 47-53
Eibl. H. and Wooley, P.: A general synthetic method for enantiomerically pure ester and ether lysophospholipids. Chem. Phys. Lipids 47 (1988) 63-68
Wooley, P. and Eibl, H.: Synthesis of enantiomerically pure phospholipids including phosphatidylserine and phosphatidylglycerol. Chem. Phys. Lipids 47 (1988) 55-62

### Beispiel 4g:

### Zwischenprodukte zur Synthese von Phospholipiden, die Zuckeralkohole im polaren Bereich enthalten.

Wie bisher beschrieben, hat die Einführung im polaren Bereich von Phospholipiden über Oligoglycerine einen ausgeprägten Effekt auf die Blutzrikulation, wenn diese Substanzen als Liposomenbestandteile eingesetzt werden. Mit gleichem Ergebnis können aber anstelle der Oligoglycerine auch Zuckeralkohole verwendet werden, z.B. Phosphorsäureester von D-Mannit, D-Lyxit und D-Threit. Diese Verbindungen können mit geeigneten Schutzgruppen (siehe dazu Schema H) auf die für Oligoglycerine beschriebene Weise in Phospholipide eingeführt werden. Bei den beschriebenen Derivaten führt die Kopplung mit Phospholipiden wieder zu einer sn-1-Verknüpfung der Phosphorsäure mit dem Zuckeralkohol.

### D-Mannit-Derivat

Aus 1.2.6.5-Diisopropyliden-D-Mannit kann durch Benzylierung in 3.4-Position und Abspaltung der Isopropyliden-Schutzgruppen 3.4-0.0-Dibenzyl-D-Mannit hergestellt werden. Nach Einführung der Isopropyliden-Schutzgruppe in 1.2-Position, Tritylierung und Benzylierung der freien OH-Gruppe erhält man nach Abspaltung der Tritylgruppe die Verbindung XV, die in den polaren Bereich von Phospholipiden eingebaut werden kann.

### D-Lyxit-Derivat

1.2-Isopropyliden-3.4-0.0-dibenyzl-D-mannit (siehe oben) wird mit Perjodsäure gespalten und mit NaBH₄ zum Alkohol XVI reduziert. Diese Verbindung kann in den polaren Bereich von Phospholipiden eingebaut werden.

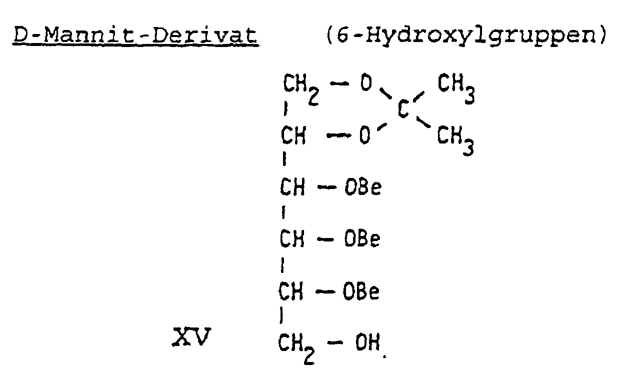

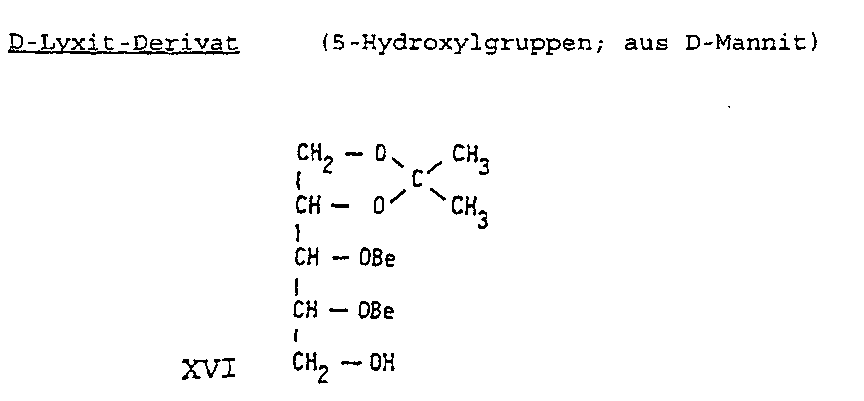

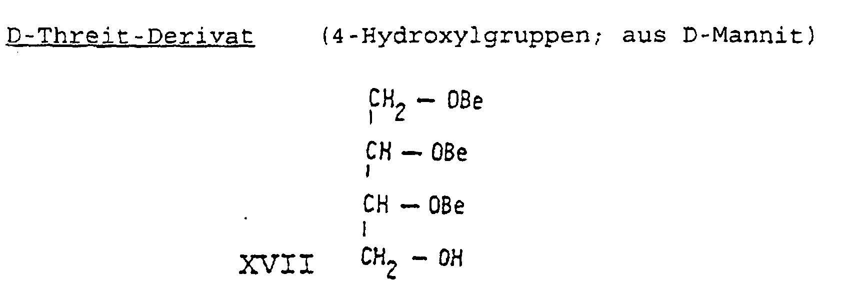

### Schema H: Polyalkohole mit mindestens 4 Hydroxylgruppen zum Einbau in den polaren Bereich von Phospholipiden

### D-Threit-Derivat

Die Verbindung XVI wird durch Benzylierung in 1.2-Isopropyliden-3.4.5-0.0.0-Tribenyzl-D-Lyxit umgewandelt. Nach Abspaltung der Isopropyliden-Schutzgruppe, Perjodsäurespaltung und Reduktion zum Alkohol erhält man XVII. Diese kann in gewohnter Weise in den polaren Bereich von Phospholipiden eingebaut werden.

### Phospholipide, die Oligoglycerine im polaren Bereich enthalten

Wir haben in früheren Publikationen beschrieben, wie auf einfache Weise Phospholipide aus Diacylglycerinen mit gesättigten und ungesättigten Fettsäureketten, mit zwei gleichen oder zwei unterschiedlichen Fettsäureketten hergestellt werden können. (DE 32 39 817 Ar; P. Woolley et al. Chem. Phys. Lipids 47 (1988) 55-62; H. Eibl et al., Chem. Phys. Lipids 47 (1988) 63-68). Entsprechend können auch Acyl/Alkyl- oder Alkyl/Acylglycerine als Ausgangsprodukt verwendet werden. Dagegen sind Phospholipide, die Dialkylglycerine enthalten, metabolisch extrem stabil und können praktisch nicht resorbiert werden.

Die genannten Verbindungen können prinzipiell nach zwei unterschiedlichen Methoden dargestellt werden. Dies ergibt sich aus der Tatsache, daß aus zwei Alkoholen, R₁-OH und R₂-OH, ein Phosphorsäurediester hergestellt werden soll.

Die Alkohole unter R₁-OH sind Alkohole, die ein Glyceringrundgerüst mit zwei Fettsäureketten und eine freie Hydroxylgruppe enthalten. Sie können aber auch nur eine Fettsäurekette und eine zusätzliche Schutzgruppe, meist Benzyl, zur Herstellung von Monoacrylphospholipiden enthalten, R₁-OH kann aber auch einen Alkohol mit einem einfachen Alkylrest mit einer oder zwei cis-Doppelbindungen darstellen.

Die Alkohole unter R₂-OH sind Alkohole, die im bisherigen Text als G₂, G₃ und G₄ bezeichnet wurden. Sie sind unter den Strukturformeln III und V (für G₂), VII und VIII (für G₃) und X bis XIV (für G₄) genannt. Entsprechend können auch die Zuckeralkoholderivate XV bis XVII eingesetzt werden.

Wie zwei Alkohole R₁-OH und R₂-OH in guten Ausbeuten zur Herstellung von Phosphorsäurediestern eingesetzt werden können, ist in Schema G beschrieben.

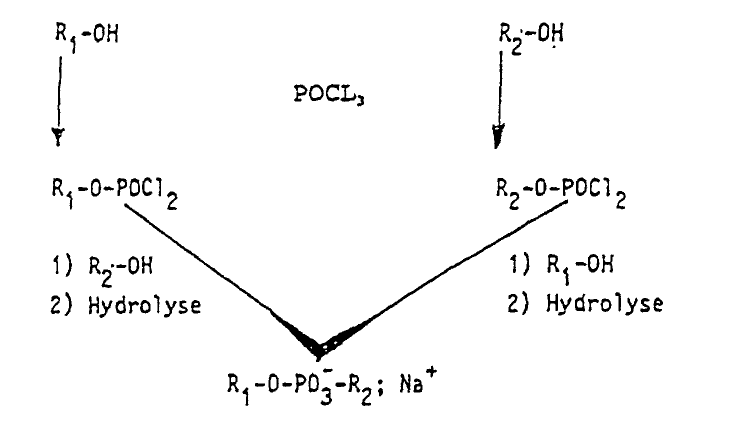

Beispielsweise ergibt sich für R₁ = 1.2-Dipalmitoyl-sn-G und R₂ = Formel XI nach Entfernung der Schutzgruppen folgende Struktur:

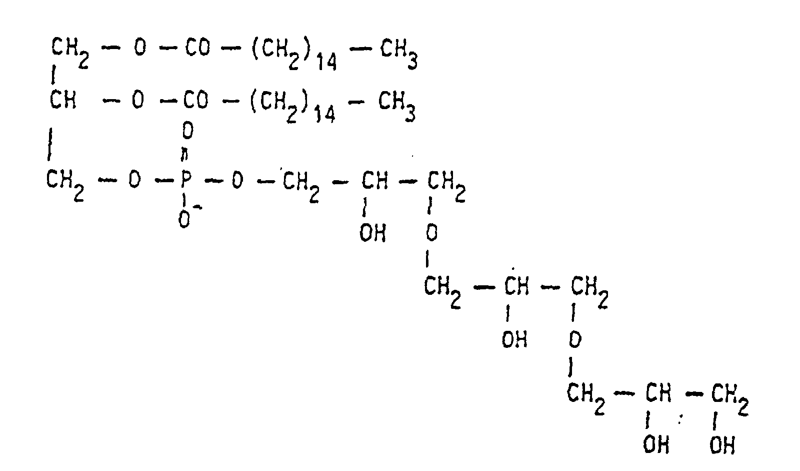

### Schema G: Phosphorsäurediester der Formel R₁O-PO⁻₃ - R₂; Na⁺

Als Phosphorylierungsmittel wird Phosphoroxychlorid eingesetzt. Aus den beiden über Phosphat zu verknüpfenden Alkoholen R₁OH oder R₂OH wird zunächst das entsprechende Phosphorsäuredichlorid hergestellt, das durch Umsetzung mit dem jeweils anderen Alkohol zum Phosphorsäuremonochlorid umgesetzt wird. Leicht basische Hydrolyse führt dann zu den Phosphorsäurediestern, die nach Abspaltung der Schutzgruppen beispielsweise in XVIII übergehen, in 1.2-Dipalmitoyl-sn-glycero-3-phospho-G₁-G₂-G₃-G₄; Na⁺-Salz.

Die in der Folge beschriebenen Beispiele können durch andere Kombination der Fettsäureketten oder durch Einführung weiterer Fettsäuren, auch synthetischer und natürlicher Herkunft, beliebig erweitert werden. Die hergestellten Phosphatidyl-Oligoglycerine können je nach Bedarf und nach Einstellung auf eine gewünschte Eigenschaft im Oligoglycerinbereich auch noch weitere Alkylketten oder auch Fettsäurereste enthalten.

Die hier vorgestellten Verfahren, basierend auf Oligoglycerinen können also in vielfältiger Weise eingesetzt und modifiziert werden, um die Eigenschaften von Liposomen zu variieren und zu beeinflussen. In Analogie zu den Hexydecylphosphocholinen den Erucylphosphocholinen sind diese Substanzen möglicherweise aber auch wichtige biologisch aktive Moleküle, die die Signaltransduktion und damit zelluläre Funktionswege beeinflussen.

### Beispiele für Phospho - G₁ - G₂- Verbindungen

| | | | |
|---|---|---|---|
| 1. | 1.2-Dipalmitoyl-sn-glycero-3-phospho-G₁-G₂; | | |
| | Na⁺-Salz: | C₄₁H₈₀NaO₁₂P | (819.04) |
| 2. | 1.2-Dimyristoyl-sn-glycero-3-phospho-G₁-G₂; | | |
| | Na⁺-Salz: | C₃₇H₇₂NaO₁₂P | (762.93) |
| 3. | 1.2-Distearoyl-sn-glycero-3-phospho-G₁-G₂; | | |
| | Na⁺-Salz: | C₄₅H₈₈NaO₁₂P | (875.14) |
| 4. | 1-Palmitoyl-2-lauroyl-sn-glycero-3-phospho-G₁-G₂; | | |
| | Na⁺-Salz: | C₃₇H₇₂NaO₁₂P | (762.93) |
| 5. | 1-Stearoyl-2-lauroyl-sn-glycero-3-phospho-G₁-G₂; | | |
| | Na⁺-Salz: | C₃₉H₇₆NaO₁₂P | (790.98) |
| 6. | 1.2-Dioleoyl-sn-glycero-3-phospho-G₁-G₂; | | |
| | Na⁺-Salz: | C₄₅H₈₄NaO₁₂P | (871.11) |
| 7. | 1.2-Dierucyl-sn-glycero-3-phospho-G₁-G₂; | | |
| | Na⁺-Salz: | C₅₃H₁₀₀NaO₁₂P | (983.32) |
| 8. | 1-Stearoyl-2-oleoyl-sn-glycero-3-phospho-G₁-G₂; | | |
| | Na⁺-Salz: | C₄₅H₈₆NaO₁₂P | (873.13) |
| 9. | 1-Palmitoyl-2-oleoyl-sn-glycero-3-phospho-G₁-G₂; | | |
| | Na⁺-Salz: | C₄₃H₈₂NaO₁₂P | (845.07) |
| 10. | 1-stearoyl-2-myristoyl-sn-glycero-3-phospho-G₁-G₂; | | |
| | Na⁺-Salz: | C₄₁H₈₀NaO₁₂P | (819.04) |
| 11. | 1-Stearoyl-2-palmitoyl-sn-glycero-3-phospho-G₁-G₂; | | |
| | Na⁺-Salz: | C₄₃H₈₄NaO₁₂P | (847.09) |
| 12. | 1-Myristoyl-sn-glycero-3-phospho-G₁-G₂; | | |
| | Na⁺-Salz: | C₂₃H₄₆NaO₁₁P | (552.57) |
| 13. | 1-Palmitoyl-sn-glycero-3-phospho-G₁-G₂; | | |
| | Na⁺-Salz: | C₂₅H₅₀Na_{O11}P | (580.62) |
| 14. | 1-Stearoyl-sn-glycero-3-phospho-G₁-G₂; | | |
| | Na⁺-Sa1z: | C₂₇H₅₄NaO₁₁P | (608.68) |
| 15. | Erucyl-phospho-G₁-G₂; | | |
| | Na⁺-Salz: | C₂₈H₅₆NaO₈P | (574.71) |
| 16. | Octadecyl-phospho-G₁-G₂; | | |
| | Na⁺-Salz: | C₂₄H₅₀NaO₈P | (520.62) |
| 17. | Hexadecyl-phospho-G₁-G₂; | | |
| | Na⁺-Salz: | C₂₂H₄₆NaO₈P | (492.56) |
| 18. | Tetradecyl-phospho-G₁-G₂; | | |
| | Na⁺-Salz: | C₂₀H₄₂NaO₈P | (464.51) |
| 19. | Oleyl-phospho-G₁-G₂; | | |
| | Na⁺-Salz: | C₂₄H₄₈NaO₈P | (518.60) |
| 20. | 1-O-Octadecyl-2-O-methyl-sn-glycero-3-phospho-G₁-G₂; | | |
| | Na⁺-Salz: | C₂₈H₅₈NaO₁₀P | (608.72) |

### Beispiele für Phospho-G₁-G₂-G₃-Verbindungen

| | | | |
|---|---|---|---|
| 1. | 1.2-Dipalmitoyl-sn-glycero-3-phospho-G₁-G₂-G₃; | | |
| | Na⁺-Salz: | C₄₄H₈₆NaO₁₄P | (893.12) |
| 2. | 1.2-Distearoyl-sn-glycero-3-phospho-G₁-G₂-G₃; | | |
| | Na⁺-Salz: | C₄₈H₉₄NaO₁₄P | (949.22) |
| 3. | 1-Palmitoyl-2-lauroyl-sn-glycero-3-phospho-G₁-G₂-G₃; | | |
| | Na⁺-Salz: | C₄₀H₇₈NaO₁₄P | (837.01) |
| 4. | 1-Stearoyl-2-lauroyl-sn-glycero-3-phospho-G₁-G₂-G₃; | | |
| | Na⁺-Salz: | C₄₂H₈₂NaO₁₄P | (865.06) |
| 5. | 1.2-Dioleoyl-sn-glycero-3-phospho-G₁-G₂-G₃; | | |
| | Na⁺-Salz: | C₄₈H₉₀NaO₁₄P | (945.19) |
| 6. | 1.2-Dierucyl-sn-glycero-3-phospho-G₁-G₂-G₃; | | |
| | Na⁺-Salz: | C₅₆H₁₀₆NaO₁₄P | (1057.40) |
| 7. | 1-Stearoyl-2-oleoyl-sn-glycero-3-phospho-G₁-G₂-G₃; | | |
| | Na⁺-Salz: | C₄₈H₉₂NaO₁₄P | (947.21) |
| 8. | 1 Palmitoyl-2-oleoyl-sn-glycero-3-phospho-G₁-G₂-G₃; | | |
| | Na⁺-Salz: | C₄₆H₈₈NaO₁₄P | (919.148) |
| 9. | 1-Stearoyl-sn-glycero-3-phospho-G₁-G₂-G₃; | | |
| | Na⁺-Salz: | C₃₀H₆₀NaO₁₃P | (682.76) |
| 10. | Erucyl-phospho-G₁-G₂-G₃; | | |
| | Na⁺-Salz: | C₃₁H₆₂NaO₁₀P | (648.79) |
| 11. | Octadecyl-phospho-G₁-G₂-G₃; | | |
| | Na⁺-Salz; | C₂₇H₅₆NaO₁₀P | (594.69) |
| 12. | Hexadecyl-phospho-G₁-G₂-G₃; | | |
| | Na⁺-Salz: | C₂₅H₅₂NaO₁₀P | (566.64) |
| 13. | 3-O-Octadecyl-2-O-methyl-sn-glycero-1-phospho-G₁-G₂-G₃; | | |
| | Na⁺-Salz: | C₃₁H₆₄NaO₁₂P | (682.80) |

### Beispiele für Phospho-G₁-G₂-G₃-G₄-Verbindungen

| | | | |
|---|---|---|---|
| 1. | 1.2-Dipalmitoyl-sn-glycero-3-phospho-G₁-G₂-G₃-G₄; | | |
| | Na⁺-Salz: | C₄₇H₉₂NaO₁₆P | (967.20) |
| 2. | 1.2-Distearoyl-sn-glycero-3-phospho-G₁-G₂-G₃-G₄; | | |
| | Na⁺-Salz: | C₅₁H₁₀₀NaO₁₆P | (1023.30) |
| 3 . | 1-Stearoyl-2-lauroyl-sn-glycero-3-phospho-G₁-G₂-G₃-G₄; | | |
| | Na⁺-Salz: | C₄₅H₈₈NaO₁₆P | (939.14) |
| 4 . | 1.2-Dioleoyl-sn-glycero-3-phospho-G₁-G₂-G₃-G₄; | | |
| | Na⁺-Salz: | C₅₁H₉₆NaO₁₆P | (1019.27) |
| 5. | 1.2-Dierucyl-sn-glycero-3-phospho-G₁-G₂-G₃-G₄; | | |
| | Na⁺-Salz: | C₅₉H₁₁₂NaO₁₆P | (1131.48) |
| 6. | 1-Stearoyl-2-oleoyl-sn-glycero-3-phospho-G₁-G₂-G₃-G₄; | | |
| | Na⁺-Salz: | C₅₁H₉₈NaO₁₆P | (1021.29) |
| 7. | Erucyl-phospho-G₁-G₂-G₃-G₄; | | |
| | Na⁺-Salz: | C₃₄H₆₈NaO₁₂P | (722.87) |

### Beispiele für Phospho-sn-G₁-Verknüpfungen

sn-1-G₁-G₂:

| | | | |
|---|---|---|---|
| 1. | 1.2-Dipylmitoyl-sn-glycero-3-phospho-sn-1-G₁-G₂; | | |
| | Na⁺-Salz: | C₄₁H₈₀NaO₁₂P | (819.04) |
| 2. | 1.2-Distearoyl-sn-glycero-3-phospho-sn-1-G₁-G₂; | | |
| | Na⁺-Salz: | C₄₅H₈₈NaO₁₂P | (875.14) |
| 3. | 1-Stearoyl-2-lauroyl-sn-glycero-3-phospho-sn-1-G₁-G₂; | | |
| | Na⁺-Salz: | C₃₉H₇₆NaO₁₂P | (790.98) |
| 4. | 1-Stearoyl-2-oleoyl-sn-glycero-3-phospho-sn-1-G₁-G₂; | | |
| | Na⁺-Salz: | C₄₅H₈₆NaO₁₂P | (873.13) |

sn-1-G₁-G₂-G₃:

| | | | |
|---|---|---|---|
| 1. | Dipalmitoyl-sn-glycero-3-phospho-sn-1-G₁-G₂-G₃; | | |
| | Na⁺-Salz: | C₄₄H₈₆NaO₁₄P | (893.12) |
| 2. | 1.2-Distearoyl-sn-glycero-3-phospho-sn-1-G₁-G₂-G₃; | | |
| | Na⁺-Salz: | C₄₈H₉₄NaO₁₄P | (949.22) |

sn-1-G₁-G₂-G₃-G₄:

| | | | |
|---|---|---|---|
| 1. | 1.2-Dipalmitoyl-sn-glycero-3-phospho-sn-1-G₁-G₂-G₃-G₄; | | |
| | Na⁺-Salz: | C₄₇H₉₂NaO₁₆P | (967.20) |
| 2. | 1.2-Distearoyl-sn-glycero-3-phospho-sn-1-G₁-G₂-G₃-G₄; | | |
| | Na⁺-Salz: | C₅₁H₁₀₀NaO₁₆P | (1023.30) |

### Beispiele für Verknüpfungen mit Zuckeralkoholen

Phospho-D-Mannit-Verbindungen

| | | | |
|---|---|---|---|
| 1. | 1.2-Dipalmitoyl-sn-glycero-3-phospho-D-mannit; | | |
| | Na⁺-Salz: | C₄₁H₈₀NaO₁₃P | (835.03) |
| 2. | 1.2-Distearoyl-sn-glycero-3-phospho-D-mannit; | | |
| | Na⁺-Salz: | C₄₅H₈₈NaO₁₃P | (891.13) |
| 3. | 1-Palmitoyl-2-lauroyl-sn-glycero-3-phospho-D-mannit; | | |
| | Na⁺-Salz: | C₃₇H₇₂NaO₁₃P | (788.92) |
| 4. | 1-Stearoyl-2-lauroyl-sn-glycero-3-phospho-D-mannit; | | |
| | Na⁺-Salz: | C₃₉H₇₆NaO₁₃P | (806.97) |
| 5. | 1-Stearoyl-2-myristoyl-sn-glycero-3-phospho-D-mannit; | | |
| | Na⁺-Salz: | C₄₁H₈₀NaO₁₃P | (835.03) |
| 6. | 1-Stearoyl-sn-glycero-3-phospho-D-mannit; | | |
| | Na⁺-Salz: | C₂₇H₅₄NaO₁₂P | (624.67) |
| 7. | Octadecyl-phospho-D-mannit; | | |
| | Na⁺-Salz: | C₂₄H₅₀NaO₉P | (536.61) |
| 8. | 1-O-Octadecyl-2-O-methyl-sn-glycero-3-phospho-D-mannit; | | |
| | Na⁺-Salz: | C₂₈H₅₈NaO₁₁P | (624.71) |

Phospho-D-Lyxit-Verbindungen

| | | | |
|---|---|---|---|
| 1. | 1.2-Dipalmitoyl-sn-glycero-3-phospho-D-lyxit; | | |
| | Na⁺-Salz: | C₄₀H₇₈NaO₁₂P | (805.00) |
| 2. | 1.2-Distearoyl-sn-glycero-3-phospho-D-lyxit; | | |
| | Na⁺-Salz: | C₄₄H₈₆NaO₁₂P | (861.10) |
| 3. | 1-Palmitoyl-2-lauroyl-sn-glycero-3-phospho-D-lyxit; | | |
| | Na⁺-Salz: | C₃₆H₇₀NaO₁₂P | (758.89) |
| 4. | 1-Stearoyl-z-lauroyl-sn-glycero-3-phospho-D-lyxit; | | |
| | Na⁺-Salz: | C₃₈H₇₄NaO₁₂P | (776.94) |
| 5. | 1-Stearoyl-2-myristoyl-sn-glycero-3-phospho-D-lyxit; | | |
| | Na⁺-Salz: | C₄₀H₇₈NaO₁₂P | (805.00) |

Phospho-D-Threit-Verbindungen

| | | | |
|---|---|---|---|
| 1. | 1.2-Dipalmitoyl-sn-glycero-3-phospho-D-threit; | | |
| | Na⁺-Salz: | C₃₉H₇₆NaO₁₁P | (774.97) |
| 2. | 1.2-Distearoyl-sn-glycero-3-phospho-D-threit; | | |
| | Na⁺-Salz: | C₄₃H₈₄NaO₁₁P | (831.07) |
| 3. | 1-Stearoyl-2-lauroyl-sn-glycero-3-phospho-D-threit; | | |
| | Na⁺-Salz: | C₃₇H₇₂NaO₁₁P | (746.91) |
| 4. | 1-Stearoyl-2-myristoyl-sn-glycero-3-phospho-D-threit; | | |
| | Na⁺-Salz: | C₃₉H₇₆NaO₁₁P | (774.97) |

### Beispiel 4h:

### Phosphorylierungsschritte (allgemeine Vorschrift) am Beispiel der Darstellung von 2.2-Dipalmitoyl-sn-glycero-3-phospho-glyceroglycerin, Na⁺-Salz

In einem 1 1 Dreihalskolben werden POCl₃ (0.1 Mol - 15.3 g) in 15 ml THF vorgelegt. Unter starkem Rühren und Eiskühlung tropft man 1.2-Dipalmitoyl-sn-glycerin (0.1 Mol - 57 g) in 100 ml THF und separat Triethylamin (0.11 Mol - 11 g) so ein, daß stets ein leichter Überschuß von Triethylamin über 1.2-Dipalmitoyl-sn-glycerin vorhanden ist, um den sich bildenden Chlorwasserstoff aufzunehmen. Die Temperatur im Reaktionsgemisch soll dabei 16 °C nicht übersteigen. Nach dem Eintropfen beläßt man noch 30 Minuten bei 16 °C und überprüft durch DC-Chromatographie die Vollständigkeit der Reaktion. (1.2-Dipalmitoyl-sn-glycerin, RF 0.8; 1.2-Dipalmitoyl-sn-glycero-3-phosphorsäuredichlorid wird durch Methanolyse in den entsprechenden Phosphorsäuredimethylester umgewandelt, Rf 0.4 in Ether.) Der zweite Phosphorylierungsschritt wird nun mit einem geschützten Oligoglycerin durchgeführt. Hier wird die Umsetzung mit 2-O-Benzyl-rac-G₁-1.3-0.0-1.2-isopropyliden-rac-G₂ beschrieben. Man tropft in das Reaktionsgemisch mit 1.2-Dipalmitoyl-sn-glycero-3-phosphorsäuredichlorid obigen Alkohol (0.105 Mol - 31 g) und Triethylamin (0.13 - 13 g) in 100 ml THF so ein, daß die Temperatur im Reaktionsgemisch 40 °C nicht übersteigt. Nach 3 Stunden bei 40 °C ist die Reaktion abgeschlossen (Ausgangsprodukt als Phosphorsäuredimethylester, Rf 0.4; Produkt als Methylester, Rf 0.7 in Ether). Man filtriert vom ausgefallenem Triethylaminhydrochlorid ab und hydrolysiert das Reaktinsgemisch, im wesentlichen 1.2-Dipalmitoyl-sn-glycero-3-phospho-2-O-benzyl-rac-glycero-1.3-0.0-1.2-isopropyliden-rac-glycerin-monochlorid neben nicht vollständig umgesetztem 1.2-Dipalmitoyl-sn-glycero-3-phosphorsäuredichlorid, mit 26 g Na₂CO₃ gelöst in 260 ml H₂O. Nach 4 Stunden wird mit 400 ml Diisopropylether versetzt und die obere Phase, die das Produkt enthält, so weit einrotiert, bis sich erste Kristalle bilden. Nun versetzt man mit 500 ml Aceton und saugt bei 20 °C die gebildeten Kristalle ab. Das Filtrat enthält das geschützte Phosphatidylglyceroglycerin, Na⁺-Salz (Rf 0.6 in CHCl₃/CH₃OH/Eisessig/H₂O 600:60:20:5). Nach Entfernen des Lösungsmittels erhält man 48 g Rohprodukt, das in 140 ml Essigsäure und 60 ml H₂O für 30 Minuten auf 60 - 70 °C erwärmt wird (Abspaltung der Isopropylidenschutzgruppe). Man versetzt mit 500 ml CHCl₃, 600 ml CH₃OH und 400 ml H₂O und schüttelt gut durch. Die untere CHCl₃-Phase wird nochmals mit 600 ml CH₃OH und 500 ml H₂O gewaschen, dabei aber so viel Na₂CO₃ zusetzen, daß der pH-Wert der wäßrigen Phase 6 erreicht. Die untere Chloroformphase wird einrotiert und der Rückstand in 400 ml THF aufgenommen. Zur Entfernung der Benzylschutzgruppe wird die Lösung mit 6 g Pd/C versetzt und in H₂-Atmosphäre debenzyliert. Die Reaktion ist nach etwa 4 Stunden beendet. Man filtriert vom Katalysator ab, entfernt das Lösungsmittel und nimmt den Rückstand (- 30 g) in 100 ml CHCl₃ auf. Man versetzt mit 900 ml Aceton und saugt die entstehenden Kristalle ab. Man erhält ein weißes Pulver, 1.2-Dipalmitoyl-sn-glycero-3-phospho-glycero-glycerin, Na⁺-Salz, Ausbeute: 26 g (∼ 32 %).

| Summenformel: C₄₁H₈₀NaO₁₂P (MG 819.04) | | | | | |
|---|---|---|---|---|---|
| ber. | C, 60.13; | H, 9.85; | Na, 2.81; | 0, 23.44; | P, 3.78 |
| gef. | C, 60.01; | H, 9.79; | Na, - ; | 0, - ; | P, 3.69 |

### 1.2-Dipalmitoyl-sn-glycero-3-phospho-glycero-glycero-glycerin, Na⁺-Salz

| Summenformel: C₄₄H₈₆NaO₁₄P (MG 893.12) | | | | | |
|---|---|---|---|---|---|
| ber. | C, 59.17; | H, 9.71; | Na, 2.57; | 0, 25.08; | P, 3.47 |
| gef. | C, 59.11; | H, 9.62; | Na, - ; | 0, - ; | P, 3.45 |

### 1.2-Dipalmitoyl-sn-glycero-3-phospho-glycero-glycero-glycerin, Na⁺-Salz

| Summenformel: C₄₇H₉₂NaO₁₆P (MG 967.20) | | | | | |
|---|---|---|---|---|---|
| ber. | C, 58.37; | H, 9.59; | Na, 2.38; | O, 26.47; | P, 3.20 |
| gef. | C, 58.29; | H, 9.53; | Na, - ; | O, - ; | P, 3.19 |

Für die Herstellung von Phosphatidyl-oligoglycerinen mit einer aliphatischen Kette, den sogenannten Lysophosphatidyl-oligoglycerinen, kann von Verbindungen ausgegangen werden, die in der sn-2-Position des Glycerins eine Benzylethergruppe tragen, z.B. 1-Palmitoyl-2-O-benzyl-sn-glycerin, 1-Stearoyl-2-O-benzyl-sn-glycerin, 1-O-Hexadecyl-2-O-benzyl-sn-glycerin, 1-O-Octadecyl-2-O-benzyl-sn-glycerin usw. Die Herstellung dieser Verbindungen haben wir in den Publikationen H. Eibl und P. Woolley, Chem. Phys. Lipids 41 (1986) 53-63 und Chem. Phys. Lipids 47 (1988) 55-62 beschrieben. Diese Verbindungen werden in der für die Herstellung von 1.2-Dipalmitoyl-sn-glycero-3-phospho-glyceroglycerin, Na⁺-Salz, beschriebenen Weise phosphoryliert und mit den geschützten Oligoglycerinen umgesetzt. Die Abspaltung der Schutzgruppen erfolgt analog. Im letzten Schritt erfolgt durch katalytische Hydrogenolyse mit Pd/C (5 % auf Aktivkohle) sowohl die Abspaltung der Benzylgruppen im Oligoglycerinbereich wie am Glycerin, das eine Acyl- oder Alkylgruppe trägt (siehe dazu die Beispiele).

Die Herstellung der Alkylphospho-oligoglycerine dagegen ist einfach, da in diesem Fall die entsprechenden Alkohole nach gegebenem Phosphorylierungsschema umgesetzt werden. Für die Darstellung der ungesättigten Vertreter muß allerdings die Tetrahydropyranylschutzgruppe anstelle der Benzylschutzgruppe verwendet werden.

Für die Synthese der ungesättigten Vertreter dieser Stoffklasse eine grundsätzlich andere Strategie beschritten werden. Hier erfolgt die Phosphorylierung von 1.2-Dibenzyl-sn-glycerin in der beschriebenen Weise (siehe dazu auch die deutsche Patentanmeldung DE 32 39 817), dann schließt sich die Umsetzung mit einem tetrahydropyranyl-geschützten Oligoglycerin an. Anstelle der Hydrolyse wird eine Methanolyse durchgeführt, wobei Phosphorsäuretriester erhalten werden, z.B. für 2-O-Tetrahydropyranyl-rac-G₁-1.3-0.0-1.2-isopropyliden-rac-G₂;

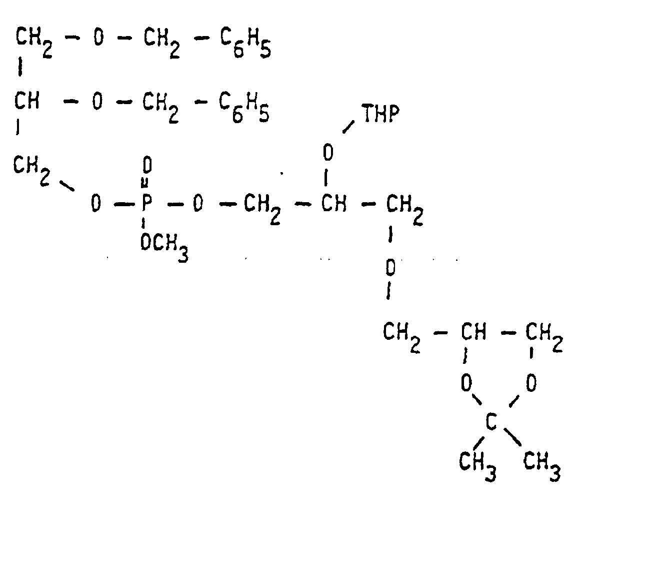

Dieses zentrale Zwischenprodukt wird nun mit Pd/C (5 % auf Aktivkohle) einer Hydrogenolyse unterworfen. Man erhält:

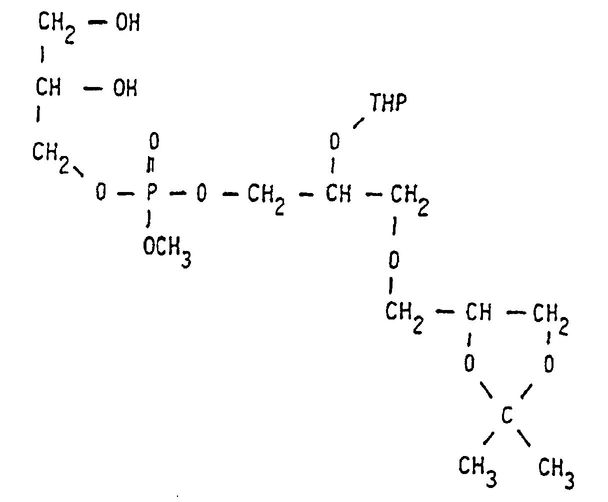

Es können nun beliebige ungesättigte und auch gesättigte Fettsäuren in die Positionen sn-1 und sn-2 des Glycerinmoleküls eingeführt werden. Danach erfolgt, wie in unserer früheren Patentanmeldung beschrieben, die Abspaltung der Methylgruppe mit LiBr und danach die Hydrolyse der Isopropyliden- und Tetrahydropyranylschutzgruppen in 70 %-iger Essigsäure bei 60 bis 70 °C. Die Dioleoylverbindungen kristallisieren schwer und müssen deshalb durch Chromatographie gereinigt werden, während die Dierucylverbindungen bereits gut in kristalliner Form erhalten werden können.

Auch zur Herstellung der gemischtkettigen Phosphatidyl-oligoglycerine ist die Phosphorsäuretriester-Strategie empfehlenswert. In diesem Falle werden wie bei der Synthese der Lysophosphatidyl-oligoglycerine die 1-Acyl-2-O-benzyl-sn-glycerine oder die 1-O-Alkyl-2-O-benzyl-sn-glycerine als Ausgangsprodukte verwendet und analog zu 1.2-Dibenzyl-sn-glycerin umgesetzt. In diesem Falle erhält man als Zwischenprodukte nach katalytischer Debenzylierung für die G₂-Verbindung:

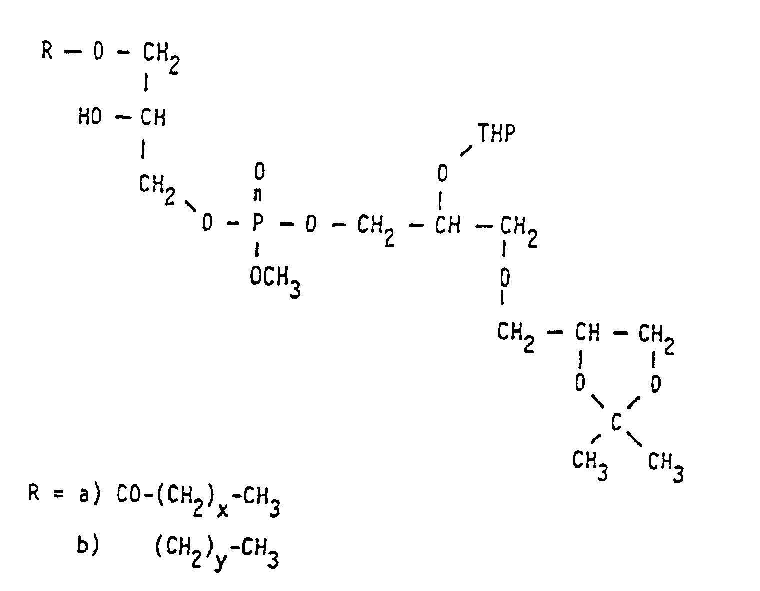

Nun können in die freie sn-2-Position ungesättigte Fettsäuren eingeführt werden und diese Moleküle wie beschrieben von ihren Schutzgruppen befreit werden. Angenehm ist, daß Moleküle mit der Fettsäurekombination 1-palmitoyl-2-oleoyl- oder 1-Stearoyl-2-oleoyl bereits gut kristallisieren.

### Beispiel 5: Weitere Eigenschaften

Mit steigender Anzahl der Glycerinmoleküle wächst die Anzahl der freien Hydroxylgruppen und damit die Polarität. Bei einem Anteil von 5 % in Wasser bildet PP-G-PG₄ als einziges PP-G-PGₙ eine klare isotrope Lösung. Die Lipide PP-G-PG₁, PP-G-PG₂ und PP-G-PG₃ lösen sich beim Erwärmen über 40 °C in Wasser und bilden Überstrukturen. Nach Unterschreiten dieser Temperatur bilden sich mit unterschiedlich ausgeprägten Hysteresen lamellare Strukturen, die durch Anisotropie (Doppelbrechung im polarisierten Licht) erkennbar sind. Die PP-G-PG₁-Lösung trübt sich am schnellsten und überschüssiges Lipid fällt nach Stehenlassen bei Raumtemperatur aus. Die lamellaren Phasen von PP-G-PG₂ und PP-G-PG₃ bleiben auch bei niedrigen Temperaturen (bis 4 °C) stabil. Während der Übergang von der isotropen in die anisotrope Phase bei Raumtemperatur für PP-G-PG₂ nach einigen Minuten erkennbar wird, erfordert dies für PP-G-PG₃ mehrere Stunden. Die Unterschiede in der Polarität äußern sich auch in verschiedenen Retentionsfaktoren (Rf-Wert) im Dünnschichtchromatogramm auf Kieseigel.

## Patentansprüche

1. Verbindung einer allgemeinen Formel (A) 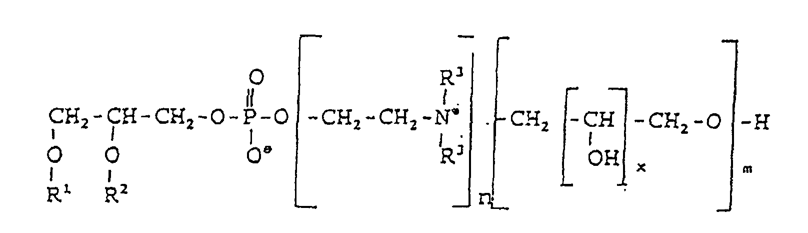 worin R¹ und R² unabhängig voneinander Wasserstoff, einen gesättigten oder ungesättigten Alkyl- oder Acylrest darstellen, der gegebenenfalls verzweigt oder/und substituiert sein kann, R³ Wasserstoff oder einen Alkylrest darstellt,
n = 0 oder 1 ist,
x eine ganze Zahl von 1 bis 4 darstellt und
m eine ganze Zahl von 2 bis 10, falls n = 0, oder eine ganze Zahl von 1 bis 10, falls n = 1, darstellt sowie 1 ist, falls x größer als 1 ist,
wobei im Falle n = 0 die Verbindung hinsichtlich des Werts von m größer als 90 % einheitlich ist, ausgenommen Verbindungen mit R¹ = Hexadecyl, R² = Methyl, n = 1, x = 1 und m = 1.

2. Verbindung nach Anspruch 1,
**dadurch gekennzeichnet,**
**dass** x = 1 ist und m eine ganze Zahl von 2 bis 5 darstellt.

3. Liposomen,
**dadurch gekennzeichnet,**
**dass** sie Phospholipide oder/und Alkylphospholipide, gegebenenfalls Cholesterin und 1 bis 50 Mol-% einer Verbindung der allgemeinen Formel (A) 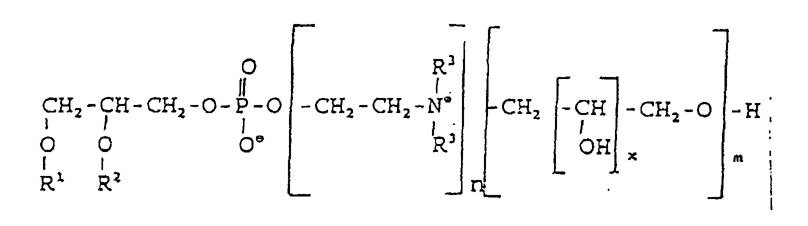 oder deren Salze enthalten, wobei das Cholesterin, die Phospholipide, die Alkylphospholipide und die Verbindung der Formel (A) zusammen 100 Mol-% ergeben, worin R¹ und R² unabhängig voneinander Wasserstoff, einen gesättigten oder ungesättigten Alkyl- oder Acylrest darstellen, der gegebenenfalls verzweigt oder/und substituiert sein kann, R³ Wasserstoff oder einen Alkylrest darstellt,
n = 0 oder 1 ist,
x eine ganze Zahl von 1 bis 4 darstellt und
m eine ganze Zahl von 2 bis 10, falls n = 0, oder eine ganze Zahl von 1 bis 10, falls n = 1, darstellt sowie 1 ist, falls x größer als 1 ist, wobei im Falle n = 0 die Verbindung (A) hinsichtlich des Werts von m größer als 90 % einheitlich ist.

4. Liposomen nach Anspruch 3,
**dadurch gekennzeichnet,**
**dass** sie 5 bis 15 Mol-% der Verbindung der Formel (A) enthalten, wobei das Cholesterin, die Phospholipide, die Alkylphospholipide und die Verbindung der Formel (A) zusammen 100 Mol-% ergeben.

5. Liposomen nach einem der Ansprüche 3 oder 4,
**dadurch gekennzeichnet,**
**dass** in Formel (A) x = 1 ist und m eine ganze Zahl von 2 bis 5 darstellt.

6. Liposomen nach einem der Ansprüche 3 bis 5,
**dadurch gekennzeichnet,**
**dass** in Formel (A) n = 0 ist.

7. Liposomen nach einem der Ansprüche 3 bis 6,
**dadurch gekennzeichnet,**
**dass** sie 25 bis 43 Mol-% Cholesterin, 5 bis 15 Mol-% einer Verbindung der allgemeinen Formel (A) und Phospholipide oder/und Alkylphospholipide enthalten.

8. Liposomen nach einem der Ansprüche 3 bis 7,
**dadurch gekennzeichnet,**
**dass** der Rest -CH₂(-CHOH)ₓ-CH₂-OH von Zuckeralkoholen stammt, die für x = 2 vier Hydroxylgruppen, für x = 3 fünf Hydroxylgruppen und für x = 4 sechs Hydroxylgruppen aufweisen.

9. Liposomen nach einem der Ansprüche 3 bis 8,
**dadurch gekennzeichnet,**
**dass** ihre Halbwertszeit in Blut mindestens 10 h beträgt.

10. Liposomen nach einem der Ansprüche 3 bis 9,
**dadurch gekennzeichnet,**
**dass** sie zusätzlich einen oder mehrere pharmazeutische Wirkstoffe enthalten.

11. Pharmazeutische Zusammensetzung,
**dadurch gekennzeichnet,**
**dass** sie Liposomen nach einem der Ansprüche 3 bis 10 und in den Liposomen eingeschlossen einen oder mehrere pharmazeutische Wirkstoffe gegebenenfalls zusammen mit pharmazeutisch üblichen Verdünnungs-, Hilfs-, Träger- und Füllstoffen enthält.

12. Verfahren zur Herstellung von Liposomen nach einem der Ansprüche 3 bis 10,
**dadurch gekennzeichnet,**
**dass** man 1 bis 50 Mol-% einer Verbindung der allgemeinen Formel (A) mit den weiteren Bestandteilen der Liposomen in einer Menge, die zusammen mit der Verbindung der Formel (A) 100 Mol-% ergibt, in eine Lipidsuspension überführt und die Lipidsuspension dann durch geeignete Maßnahmen auf an sich bekannte Weise in Liposomen überführt.

13. Verfahren zur Herstellung einer pharmazeutischen Zubereitung nach Anspruch 11,
**dadurch gekennzeichnet,**
**dass** man nach einem Verfahren gemäß Anspruch 12 arbeitet und zum Einschluss wasserunlöslicher Wirkstoffe den Wirkstoff zusammen mit den Lipidbestandteilen löst und zum Einschluss wasserlöslicher Wirkstoffe den Lipidfilm mit einer wässrigen Lösung versetzt, die den wasserlöslichen Wirkstoff enthält.

14. Verfahren zur Herstellung einer Verbindung der allgemeinen Formel 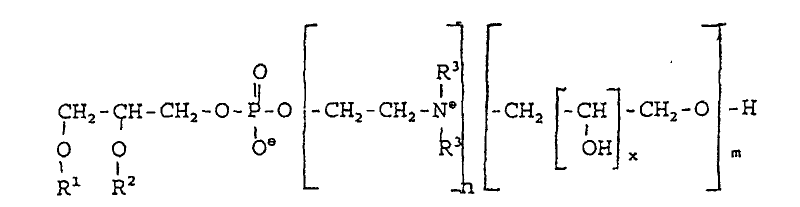 worin R¹ und R² unabhängig voneinander Wasserstoff, einen gesättigten oder ungesättigten Alkyl- oder Acylrest darstellen, der gegebenenfalls verzweigt oder/und substituiert sein kann, R³ Wasserstoff oder einen Alkylrest darstellt,
n = 0 oder 1 ist,
X eine ganze Zahl von 1 bis 4 darstellt und
m eine ganze Zahl von 2 bis 10, falls n = 0, oder eine ganze Zahl von 1 bis 10, falls n = 1, darstellt sowie 1 ist, falls x größer als 1 ist,
wobei im Falle n = 0 die Verbindung hinsichtlich des Wertes von m größer als 90 % einheitlich ist,
**dadurch gekennzeichnet,**
**dass** ein definiertes Oligoglycerin oder ein C₄-C₆-Zuckeralkohol unter Verwendung eines Phosphorylierungsmittels an einen Alkohol der Formel CH₂OR¹-CHOR²-CHOH angehängt wird.

15. Geschütztes Oligoglycerin der Formel (B) 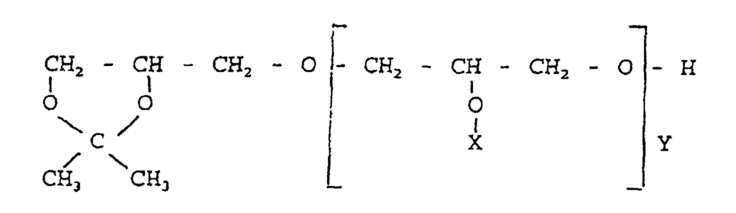 worin Y eine ganze Zahl von 1 bis 9 und X eine Benzyl-, Alkyl- oder Tetrahydropyranylgruppe darstellt, ausgenommen = Methyl wenn Y = 1 und X = CH₃ ist.

16. Alkyloligoglycerin der Formel (C) 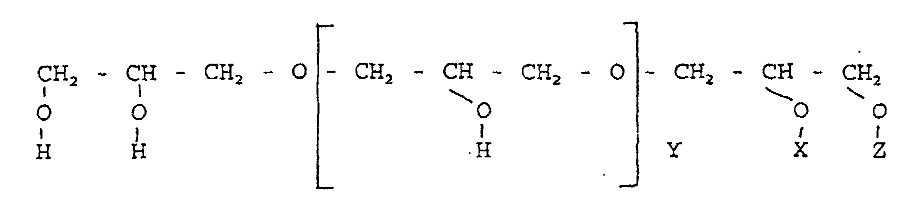 worin Y eine ganze Zahl von 0 bis 8 darstellt und einer der Reste X oder Z einen gesättigten oder ungesättigten Alkylrest und der andere der Reste Wasserstoff darstellt, ausgenommen Y = 0, wenn X = Methyl oder einer der Reste X oder Z C₁₆H₃₃ ist.

17. Liposomen,
**dadurch gekennzeichnet,**
**dass** sie Phospholipide oder/und Alkylphospholipide, gegebenenfalls Glycerin und 1 bis 50 Mol-% einer nach Anspruch 14 erhaltenen Verbindung enthalten.

## Claims

1. A compound with the general formula (A) 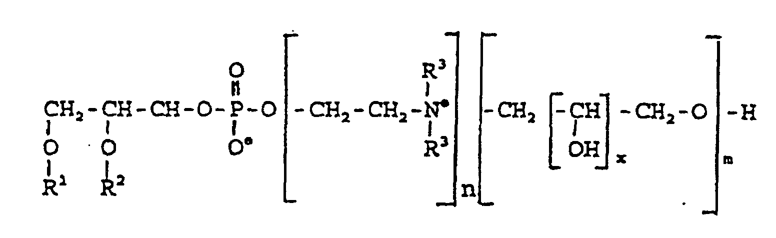 where R¹ and R², independent of each other, stand for hydrogen or a saturated or unsaturated alkyl or acyl residue, which may be branched and/or substituted, R³ stands for hydrogen or an alkyl residue,
n = 0 or 1,
x is a whole number from 1 to 4 and
m is a whole number from 2 to 10 if n = 0, or a whole number from 1 to 10 if n = 1, or 1 if x is greater than 1,
and where, in the case that n = 0, the compound is more than 90 % uniform with respect to the value of m, except compounds in which R¹ = hexadecyl, R² = methyl, n= 1, x = 1 and m = 1.

2. The compound of claim 1,
**characterised in that**
x = 1 and m is a whole number from 2 to 5.

3. Liposomes,
**characterised in that**
they contain phospholipids and/or alkyl phospholipids, maybe cholesterol, and 1 to 50 mol % of a compound with the general formula (A), 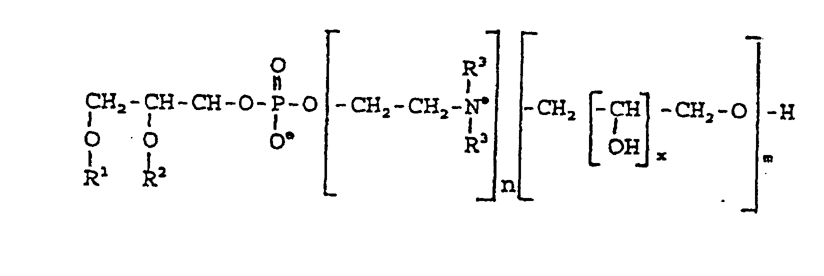 or salts thereof, where the cholesterol, the phospholipids, the alkyl phospholipids and the compound of formula (A) together make up 100 mol %, and R¹ and R², independent of each other, stand for hydrogen, a saturated or unsaturated alky or acyl residue which may be branched and/or substituted,
R³ stands for hydrogen or an alkyl residue,
n = 0 or 1,
x is a whole number from 1 to 4 and
m is a whole number from 2 to 10 if n = 0, or a whole number from 1 to 10 if n=1, or 1 if x is greater than 1, and where, in the case that n = 0, the compound (A) is more than 90 % uniform with respect to the value of m.

4. Liposomes according to claim 3,
**characterised in that**
they contain 5 to 15 mol % of the compound of formula (A), with the cholesterol, the phospholipids, the alkyl phospholipids and the compound of formula (A) together making up 100 mol %.

5. Liposomes according to one of claims 3 or 4,
**characterised in that,**
in formula (A), x = 1 and m is a whole number from 2 to 5.

6. Liposomes according to one of claims 3 to 5,
**characterised in that,**
in formula (A), n = 0.

7. Liposomes according to one of claims 3 to 6,
**characterised in that**
they contain 25 to 43 mol % cholesterol, 5 to 15 mol % of a compound of general formula (A), and phospholipids and/or alkyl phospholipids.

8. Liposomes according to one of claims 3 to 7,
**characterised in that**
the residue -CH₂(-CHOH)ₓ-CH₂-OH derives from sugar alcohols which have four hydroxyl groups for x = 2, five hydroxyl groups for x = 3 and six hydroxyl groups for x = 4.

9. Liposomes according to one of claims 3 to 8,
**characterised in that**
their half-life in blood is at least 10 h

10. Liposomes according to one of claims 3 to 9,
**characterised in that**
they additionally contain one or more pharmaceutical active ingredients.

11. A pharmaceutical composition,
**characterised in that**
it contains liposomes according to one of claims 3 to 10 and, entrapped in the liposomes, one or more pharmaceutical active ingredients, combined if necessary with standard pharmaceutical diluents, adjuvants, carrier media and fillers.

12. A method of preparing liposomes according to one of claims 3 to 10,
**characterised in that**
1 to 50 mol % of a compound of general formula (A), together with the other components of the liposomes in a quantity which, together with the compound of formula (A), makes up 100 mol %, are transformed into a lipid suspension and the lipid suspension, in turn, is transformed into liposomes by means of suitable measures in a manner known per se.

13. A method of preparing a pharmaceutical formulation as claimed in claim 11,
**characterised in that**
one proceeds according to the method of claim 12, water-insoluble active ingredients being entrapped by dissolving the active ingredient together with the lipid constituents, and water-soluble active ingredients being entrapped by adding an aqeous solution containing the water-soluble active ingredient to the lipid film.

14. A method of preparing a compound with the general formula (A) 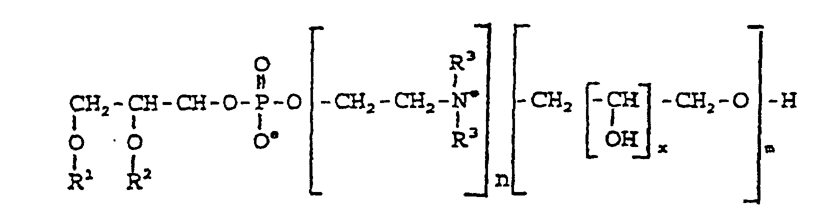 where R¹ and R², independent of each other, stand for hydrogen or a saturated or
unsaturated alkyl or acyl residue, which may be branched and/or substituted, R³ stands for hydrogen or an alkyl residue,
n = 0 or 1,
x is a whole number from 1 to 4 and
m is a whole number from 2 to 10 if n = 0, or a whole number from 1 to 10 if n = 1, or 1 if x is greater than 1,
and where, in the case that n = 0, the compound is more than 90 % uniform with respect to the value of m, ,
**characterised in that**
a defined oligoglycerol or a C₄-C₆ sugar alcohol is linked with an alcohol of the formula CH₂OR¹-CHOR²-CHOH through use of a phosphorylation agent.

15. A protected oligoglycerol with the formula (B), 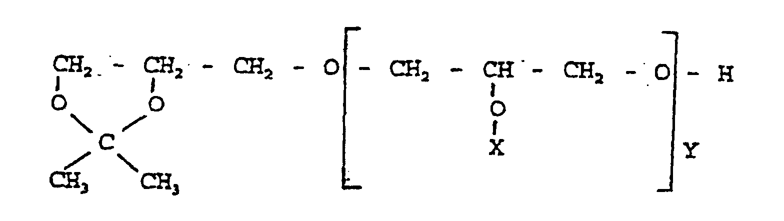 in which Y is a whole number from 1 to 9 and X is a benzyl, alkyl or tetrahydropyranyl group, except X= methyl if Y = 1.

16. An alkyl oligoglycerol with the formula (C) 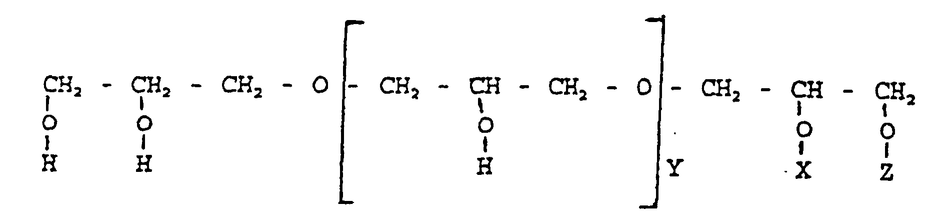 in which Y is a whole number from 0 to 8 and one of the residues X or Z is a saturated or unsaturated alkyl residue, while the other residue is hydrogen, except Y = 0 if X = methyl or one of the residues X or Z is C₁₆H₃₃.

17. Liposomes
**characterised in that**
they contain phospholipids and/or alkyl phospholipids, maybe glycerol and 1 to 50 mol % of a compound obtained according to the method of claim 14.

## Revendications

1. Composé de formule générale (A) 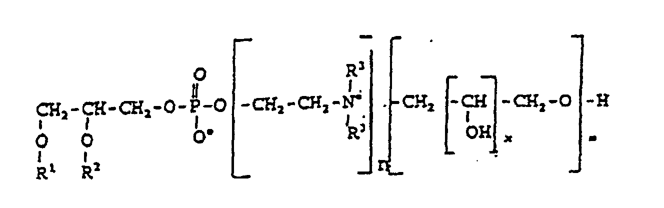 dans laquelle R¹ et R² représentent indépendamment l'un de l'autre l'hydrogène, un radical alkyle ou acyle pouvant être saturé ou non saturé, éventuellement ramifié ou/et substitué, R³ l'hydrogène ou un radical alkyle,
n = 0 ou 1,
x représente un nombre entier de 1 à 4 et
m représente un nombre entier de 2 à 10, si n = 0, ou un nombre entier de 1 à 10, si n = 1, et 1 si x est plus grand que 1,
dans lequel dans le cas où n = 0, le composé est selon la valeur de m à plus de 90 % homogène, à l'exception des composés avec R¹ = hexadécyle, R² = méthyle, n = 1, x = 1 et m = 1.

2. Composé selon la revendication 1,
**caractérisé par le fait**
**que** x = 1 et que m représente un nombre entier de 2 à 5.

3. Liposomes
**caractérisés par le fait**
**qu'**ils contiennent des phospholipides ou/et des alkylphospholipides, éventuellement du cholestérol et 1 à 50 moles % d'un composé de formule générale (A) 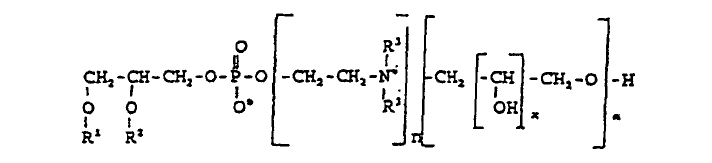 ou de ses sels, le cholestérol, les phospholipides, les alkylphospholipides et le composé de formule (A) formant ensemble 100 moles %, dans laquelle R¹ et R² représentent indépendamment l'un de l'autre l'hydrogène, un radical alkyle ou acyle pouvant être saturé ou non saturé, éventuellement ramifié ou/et substitué, R³ l'hydrogène ou un radical alkyle,
n = 0 ou 1,
x représente un nombre entier de 1 à 4
m représente un nombre entier de 2 à 10, si n = 0, ou un nombre entier de 1 à 10, si n = 1, et 1 si x est plus grand que 1, dans lequel dans le cas où n = 0, le composé (A) est selon la valeur de m à plus de 90 % homogène.

4. Liposomes selon la revendication 3,
**caractérisés par le fait**
**qu'**ils contiennent de 6 à 15 moles % du composé de formule (A), le cholestérol, les phospholipides, les alkylphospholipides et le composé de formule (A) formant ensemble 100 moles %.

5. Liposomes selon une des revendications 3 ou 4
**caractérisés par le fait**
**que** dans la formule (A) x = 1 et que m représente un nombre de 2 à 5.

6. Liposomes selon une des revendications 3 à 5
**caractérisés par le fait**
**que** dans la formule (A) n = 0.

7. Liposomes selon une des revendications 3 à 6
**caractérisés par le fait**
**qu'**ils contiennent de 25 à 43 moles % de cholestérol, de 5 à 15 moles % d'un composé de formule générale (A) et des phospholipides ou/et des alkylphospholipides.

8. Liposomes selon une des revendications 3 à 7
**caractérisés par le fait**
**que** le radical CH₂(-CHOH)ₓ-CH₂-OH est issu d'alcools de sucre qui présentent quatre groupes hydroxyles pour x = 2, cinq groupes hydroxyles pour x = 3 et six groupes hydroxyles pour x = 4.

9. Liposomes selon une des revendications 3 à 8
**caractérisés par le fait**
**que** leur temps de demi-vie sanguine s'élève à au moins 10 h.

10. Liposomes selon une des revendications 3 à 9
**caractérisés par le fait**
**qu'**ils contiennent un ou plusieurs principes pharmaceutiques actifs.

11. Composition pharmaceutique
**caractérisée par le fait**
**qu'**elle contient des liposomes selon une des revendications 3 à 10 et que soient inclus dans les liposomes un ou plusieurs principes pharmaceutiques actifs avec les diluants, les excipients, les supports et les agents de charge pharmaceutiques usuels.

12. Procédé de fabrication de liposomes selon une des revendications 3 à 10,
**caractérisé par le fait**
**que** l'on convertit en suspension lipidique 1 à 50 moles % d'un composé de formule générale (A) ainsi que les autres composants des liposomes dans une quantité qui avec le composé de formule (A) donne 100 moles %, et qu'on'convertit ensuite cette suspension lipidique en liposomes à l'aide de mesures appropriées bien connues.

13. Procédé de fabrication d'une préparation pharmaceutique selon la revendication 11,
**caractérisé par le fait**
**que** l'on travaille après l'application d'un procédé selon la revendication 12 et que, pour inclure des principes actifs non solubles dans l'eau, l'on dissout le principe actif et les parties lipidiques et que, pour inclure des principes actifs solubles dans l'eau, on mélange le film lipidique à une solution aqueuse contenant le principe actif soluble dans l'eau.

14. Procédé de fabrication d'un composé de formule générale 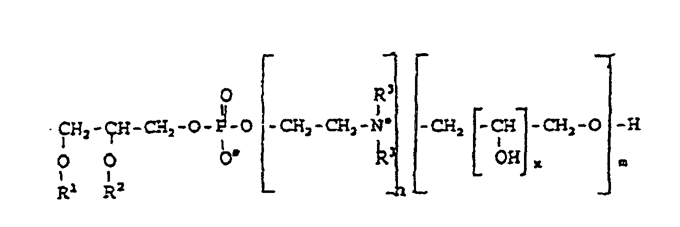 dans laquelle R¹ et R² représentent indépendamment l'un de l'autre l'hydrogène, un radical alkyle ou acyle pouvant être saturé ou non saturé, éventuellement ramifié ou/et substitué, R³ l'hydrogène ou un radical alkyle,
n = 0 ou 1,
x représente un nombre entier de 1 à 4
m représente un nombre entier de 2 à 10 si n = 0, ou un nombre entier de 1 à 10 si n = 1, et 1 si x est plus grand que 1,
dans lequel dans le cas où n = 0, le composé est selon la valeur de m à plus de 90 % homogène,
**caractérisé par le fait**
**qu'**une oligoglycérine définie ou un alcool de sucre en C₄ à C₆ est ajouté à un alcool de formule CH²OR¹-CHOR²-CHOH en utilisant un agent de phosphorylation.

15. Oligoglycérine protégée de formule (B) 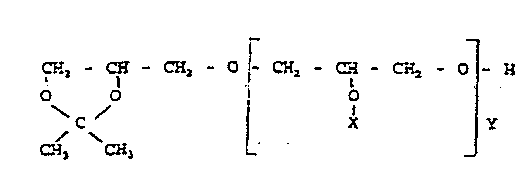 dans laquelle Y représente un nombre entier de 1 à 9 et X un groupe benzyle, alkyle ou tetrahydropyrényle à l'exception X = méthyle lorsque Y = 1.

16. Alkyloligoglycérine de formule (C) 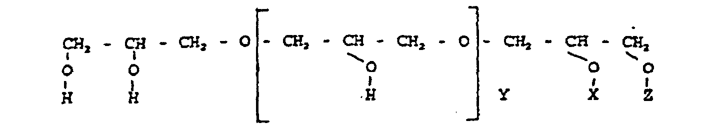 dans laquelle Y représente un nombre entier de 0 à 8 et où un des radicaux X ou Z représente un reste alkyle saturé ou non saturé et l'autre radical représente l'hydrogène, à l'exception de Y = 0, lorsque X = méthyle ou qu'un des radicaux X ou Z est le C₁₆H₃₃.

17. Liposomes
**caractérisés par le fait**
**qu'**ils contiennent des phospholipides ou/et des alkylphospholipides, éventuellement de la glycérine et de 1 à 50 moles % d'un composé obtenu selon la revendication 14.
